# EUROPEAN PATENT APPLICATION

(11) **EP 4 596 570 A1**
(43) Date of publication of application: **06.08.2025**
(21) Application number: 24382104.8
(22) Date of filing: 01.02.2024
(51) Int. Cl.: C07K 5/00, C07K 7/00, C07K 14/00, A61K 47/50, A61P 35/00

(54) **SRC INHIBITORS AND USES THEREOF**

(71) Applicant: Universitat de Barcelona, 08028 Barcelona (ES); Universitat Internacional De Catalunya, Fundació Privada, 08017 Barcelona (ES)
(72) Inventor: PONS VALLÈS, Miquel, 08028 Barcelona (ES); REYES FARIAS, Marjorie Grace, 08028 Barcelona (ES); MEZQUITA MAS, Betlem, 08017 Barcelona (ES)
(74) Representative: Hoffmann Eitle

(57) **Abstract**

The present invention relates to the chemical and medical field, in particular to the synthesis of new molecules by click chemistry with anticancer properties. More specifically, the present invention relates to new compounds, pharmaceutical compositions comprising said compounds and their use in therapy, such as in cancer therapy.

## Description

### FIELD OF INVENTION

The present invention relates to the chemical and medical field, in particular to the synthesis of new molecules by click chemistry with anticancer properties. More specifically, the present invention relates to new compounds, pharmaceutical compositions comprising said compounds and their use in therapy, such as in cancer therapy.

### BACKGROUND OF THE INVENTION

Cancer is the leading cause of death in men and the second in women in Spain. Metastases and the acquisition of resistance to treatments are directly related to cancer lethality. The protein Src is implied in both processes and high Src activity is associated to bad prognosis in colorectal, prostate, lung and breast cancers. High levels of Src are also found in pancreatic cancers, head and neck cancer and cancers of the nervous system, melanoma, and other tumor types. Thus Src is a validated drug target for cancer.

Currently there are four Src inhibitors approved by FDA which are also inhibitors of other kinases and are primarily designated for treating hematological malignancies, such as chronic myeloid leukemia or acute lymphoblastic leukemia: dasatinib, bosutinib, vandetanib and ponatinib. All of them target the kinase binding domain and have failed to be effective for solid tumors. Possible reasons for this failure could be off-target effects, as Src inhibitors often inhibit other kinases that have similar binding pockets. This leads to toxic side effects and limits dosage. Another reason for the failure could be the pathological effect of inhibiting physiological functions of Src in normal cells. Src is expressed in all tissues with enhanced expression in brain, osteoclasts and platelets.

The inventors sought to find a way of selectively inhibiting Src in cancer cells by targeting the intrinsically disordered region of Src (residues 1-86 comprising the SH4 and Unique domains) that is believed to contribute to the cell-type specific activity of Src and its differential behavior in normal and cancer cells. The biological activity of the N-terminal region of c-Src has been demonstrated by point mutations that result in a 50% decrease of the Src-dependent tumor growth in a colorectal cancer cell model.

Intrinsically disordered regions of proteins are usually considered undruggable because they lack of a well-defined three-dimensional structure to which candidate drugs may bind. On the other hand, the lack of structure suggests that short peptides with sequences present in the target protein may mimic relevant properties of the disordered regions. This approach has been precedented by the use of a short peptide with a sequence derived from the Unique domain of Src to inhibit neuropathic pain in mice, but not in cancer related applications. [X M Yu , R Askalan, G J Keil nd, M WSalter. PMID: 9005855 DOI: 10.1126/science.275.5300.674]. However, the use of peptides as drugs for intracellular targets requires the use of cell penetrating fusion peptides or alternative intracellular cell delivery methods and is limited by the sensitivity of peptides to degradation by proteases. An alternative approach, described in this invention, is based on binding of the relevant peptides to a non-peptidic hydrophobic backbone. The use of the click reaction Cu(I)catalyzed azide-alkyne cycloaddition (CuAAC) facilitates the access to these hybrid compounds.

Click chemistry encompasses group of reactions that are rapid, simple to perform, require none or almost no purification, versatile, regiospecific and render high product yields (Hein CD, Liu XM, Wang D. Click chemistry, a powerful tool for pharmaceutical sciences. Pharm Res. 2008;25(10):2216-2230. doi:10.1007/s11095-008-9616-1). CuAAC is one of the most extensively used click reactions mostly to attach labels or to purify proteins that have reacted with probes containing either azide or alkyne groups. A different type of click reaction, called sulfur (VI) fluoride exchange, has been used to directly target nucleofilic sites in the active site of protein kinase Src-family proteins (Gushwa NN, Kang S, Chen J, Taunton J. Selective targeting of distinct active site nucleophiles by irreversible SRC-family kinase inhibitors. J Am Chem Soc. 2012;134(50):20214-20217. doi:10.1021/ja310659j; Barrow AS , Smedley CJ , Zheng Q , Li S , Dong J , Moses JE. The growing applications of SuFEx click chemistry. Chem Soc Rev. 2019;48(17):4731-4758. doi:10.1039/c8cs00960k).

Some small molecules, non-peptidic, 1,2,3-triazole- containing molecules prepared by click chemistry have shown weak Src kinase inhibitory activity in vitro (Lebeau A, Abrioux C, Bénimèlis D, Benfodda Z, Meffre P. Synthesis of 1,4-disubstituted 1,2,3-triazole Derivatives Using Click Chemistry and their Src Kinase Activities J. Med Chem. 2016;13(1):40-48. doi: 10.2174/1573406412666160404125718; Kumar D, Reddy VB, Kumar A, Mandal D, Tiwari R, Parang K. Click chemistry inspired one-pot synthesis of 1,4-disubstituted 1,2,3-triazoles and their Src kinase inhibitory activity. Bioorganic & Medicinal Chemistry Letters 21 (2011) 449-452. doi:10.1016/j.bmcl.2010.10.121).

### SUMMARY OF THE INVENTION

The inventors have shown that compounds with the formula (I) composed by two identical peptides based on the Src sequence linked to a hydrophobic, rigid, non-peptidic framework have the capacity to inhibit the proliferation and migration of cancer cells and, therefore, could be potentially used as anticancer drugs.

The sequence of the disordered region of Src is

MGSNKSKPKDASQRRRSLEPAENVHGAGGGAFPASQTPSKPASADGHRGPSAAFAPAAA
EPKLFGGFNSSDTVTSPQRAGPLAGGV (SEQ ID NO: 2)

The region shown in italics is the SH4 domain, acting as a membrane anchoring site. The inventors have generated the sequence SLE bound to the hydrophobic framework by a 1,4-substituted 1,2,3-triazole ring generated by click chemistry with 2-ethynylpyrrolidone as mimic of the C-terminal proline. This compound was surprisingly able to reduce the number of colonies in Src-expressing SW620 colorectal cancer cells (Fig. 11 A and B) and inhibit the capacity of proliferation and migration in wound healing assays (Fig. 12). The compound of the invention failed to show toxicity in SW620 cells as depicted by viability assays (Fig. 13).

The inventors also demonstrated that the combination of the proline mimic with the next hydrophobic residue (after leucine) in the sequence of the Unique domain, V, displayed similar anti-tumor properties and toxicity as the SLE sequence.

The other sequence tested, that gave no activity, was the LK located next to the Unique Lipid Binding Region, far from the SH4 domain. Confirming that the 1,2,3-triazole linked to the hydrophobic framework is not responsible for the observed activity. Thus, the sequences that efficiently targeted tumor cells, SLE and V, contain sequences with a least a hydrophobic residue of the Unique domain of Src near the SH4 lipid binding region.

A possible explanation for this preference is the fact that Src binds to membranes by the SH4 domain and, subsequently, undergoes membrane associated oligomerization. Therefore, by anchoring duplicated Src sequences close to the membrane anchoring site displayed by a rigid hydrophobic framework, we may be interfering with Src signaling.

Accordingly, a first aspect of the invention relates to a compound of formula (I): 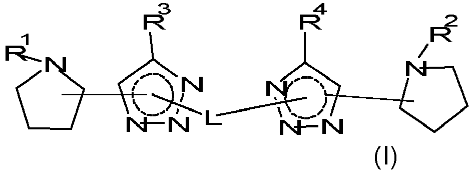
a pharmaceutically salt or isomer thereof,
wherein
R¹ and R² are the same or different and are selected from the group consisting of: a non-polar amino acid and a peptide, wherein
   - the non-polar amino acid or peptide:
      ∘ is N-terminal protected,
      ∘ binds to the N atom of the pyrrolidinyl ring by its C-terminal end, forming an amide bond, and
      ∘ the lateral chain of the one or more amino acid(s) is optionally protected;
   - the peptide comprises sequence SEQ ID NO: 1 (MGSNKSKPKDASQRRRSLEPAENVHGAGGG) or a fragment thereof;
R³ and R⁴ are the same or different and represent hydrogen, (C₁-C₅)alkyl, (C₂-C₅)alkenyl, (C₂-Cs)alkynyl, (C₁-C₅)alkyl substituted by one or more substituents Su₄, (C₂-C₅)alkenyl substituted by one or more substituents Su₅, or (C₂-C₅)alkynyl substituted by one or more substituents Su₆; and
L is of formula (II): 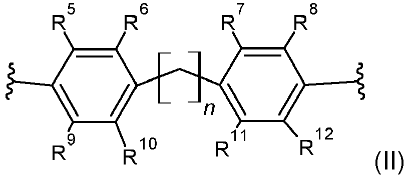
wherein
n is an entire number selected from 0 to 3, wherein when n=O, both rings are linked by a single bond;
R⁵ to R¹² are the same or different and are independently selected from the group consisting of: H, halogen, (C₁-C₁₀)alkyl, NO₂, OR¹³, SR¹⁴, NR¹⁵R¹⁶, and (+)NR¹⁷R¹⁸R¹⁹; or, alternatively,
R⁵ and R⁸ to R¹² are as defined above, and R⁶ and R⁷, together with the carbon atoms to which they are attached, form a ring system; or, alternatively,
R⁵ to R⁹ and R¹² are as defined above, and R¹⁰ and R¹¹ form, together with the carbon atoms to which they are attached, a ring system; or, alternatively,
R⁵, R⁸, R⁹ and R¹² are as defined above, R⁶ and R⁷ together with the carbon atoms to which they are attached form a ring system, and R¹⁰ and R¹¹ together with the carbon atoms to which they are attached form another ring system;
wherein the term "ring system" means a ring having from 6 to 10 members selected from CRₓ, C(RₓR_{y}), O, S, N, or NR_{y};
R¹³ and R¹⁴ represent hydrogen, (C₁-C₁₀)alkyl or (C₁-C₁₀) alkyl substituted by one or more substituents Su₇;
R¹⁵ to R¹⁹ are the same or different and are independently selected from the group consisting of: H, (C₁-C₁₀)alkyl, and (C₁-C₁₀)alkyl substituted by one or more substituents Su₈;
Rₓ and R_{y} represent hydrogen, (C₁-C₁₀)alkyl or (C₁-C₁₀) alkyl substituted by one or more substituents Su₉; and
Su₁ to Su₉ are independently selected from the group consisting of: halogen, cyano, nitro, (C₁-C₆)haloalkyl, (C₁-C₆)alkyl, (C₁-C₆)hydroxyalkyl, and (C₁-C₆)alkoxy.

The fragments of SEQ ID NO: 1 are defined as described later in the description.

In a second aspect, the present invention provides a pharmaceutical composition comprising a therapeutically effective amount of compounds of formula as defined in any of the preceding embodiments together with one or more pharmaceutically acceptable excipients and/or carriers.

The third aspect of the invention relates to a compound described in any of the preceding embodiments for use in therapy.

The fourth aspect of the invention relates to a compound described in any of the preceding embodiments for use in the treatment of cancer, wherein there is an activation of Src in said cancer.

### BRIEF DESCRIPTION OF THE DRAWINGS

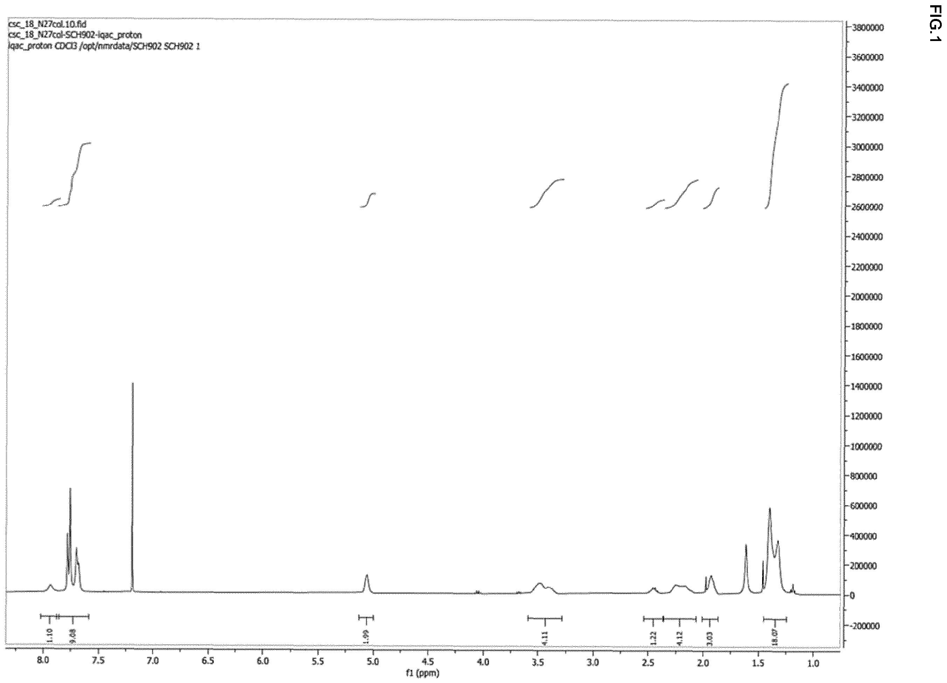

**Figure 1.** ¹H-MR spectrum of (2S,2'S)-di-tert-butyl 2,2'-(1,1'-([1,1'-biphenyl]-4,4'-diyl)bis(1H-1,2,3-triazole-4,1-diyl))bis(pyrrolidine-1-carboxylate).

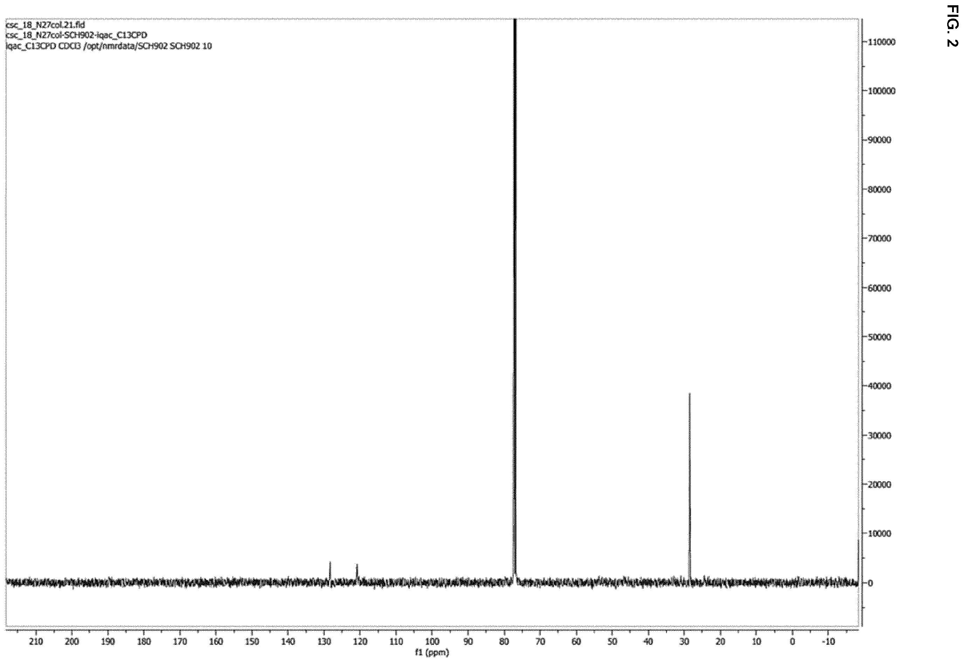

**Figure 2****.** ¹³C-NMR spectrum of (2S,2'S)-di-tert-butyl 2,2'-(1,1'-([1,1'-biphenyl]-4,4'-diyl)bis(1H-1,2,3-triazole-4,1-diyl))bis(pyrrolidine-1-carboxylate).

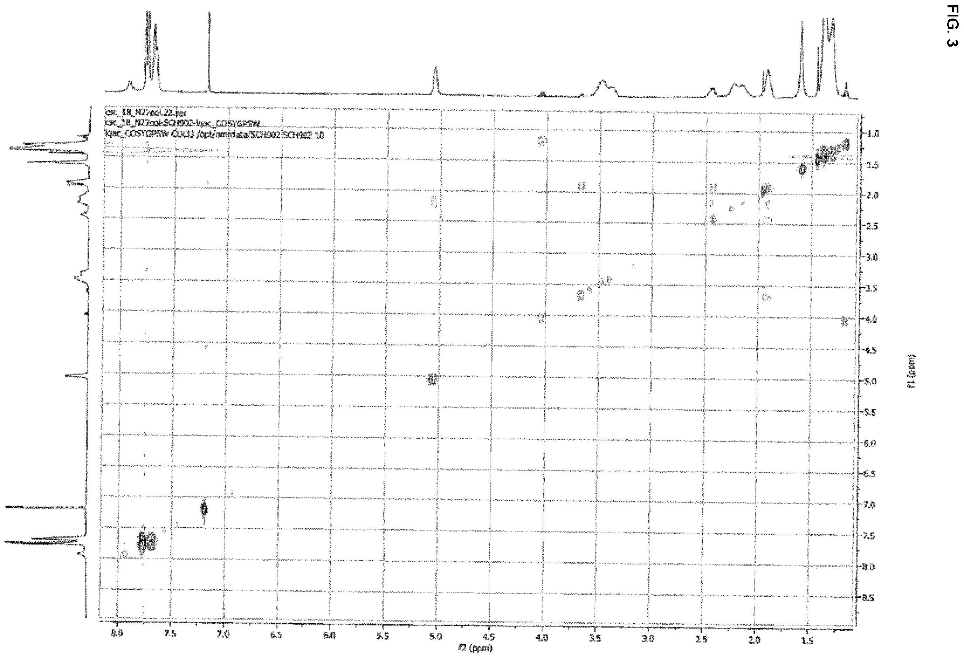

**Figure 3****.** 2D-COSY spectrum of (2S,2'S)-di-tert-butyl 2,2'-(1,1'-([1,1'-biphenyl]-4,4'-diyl)bis(1H-1,2,3-triazole-4,1-diyl))bis(pyrrolidine-1-carboxylate).

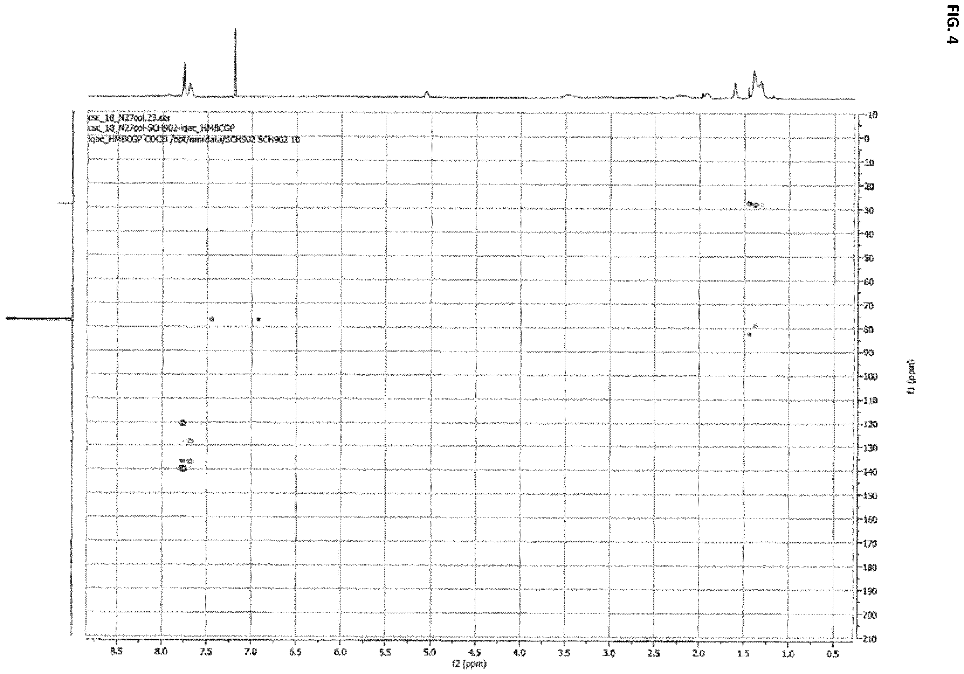

**Figure 4****.** D-HMBC spectrum of (2S,2'S)-di-tert-butyl 2,2'-(1,1'-([1,1'-biphenyl]-4,4'-diyl)bis(1H-1,2,3-triazole-4,1-diyl))bis(pyrrolidine-1-carboxylate).

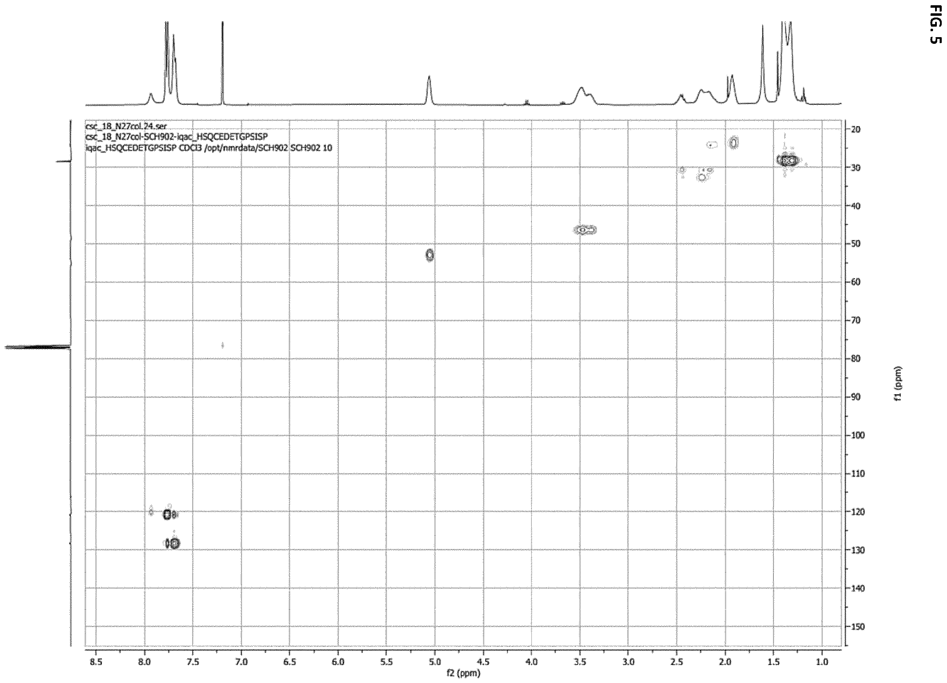

**Figure 5****.** 2D 1H-13C-HSQC spectrum of (2S,2'S)-di-tert-butyl 2,2'-(1,1'-([1,1'-biphenyl]-4,4'-diyl)bis(1H-1,2,3-triazole-4,1-diyl))bis(pyrrolidine-1-carboxylate).

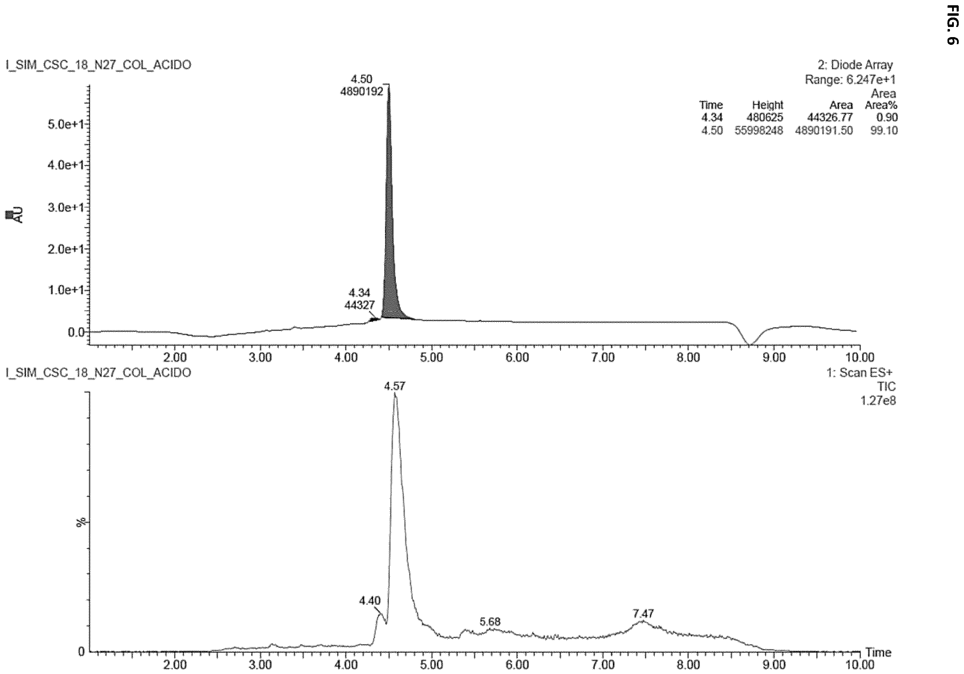

**Figure 6****.** C-18 reverse phase HPLC characterization of (2S,2'S)-di-tert-butyl 2,2'-(1,1'-([1,1'-biphenyl]-4,4'-diyl)bis(1H-1,2,3-triazole-4,1-diyl))bis(pyrrolidine-1-carboxylate).

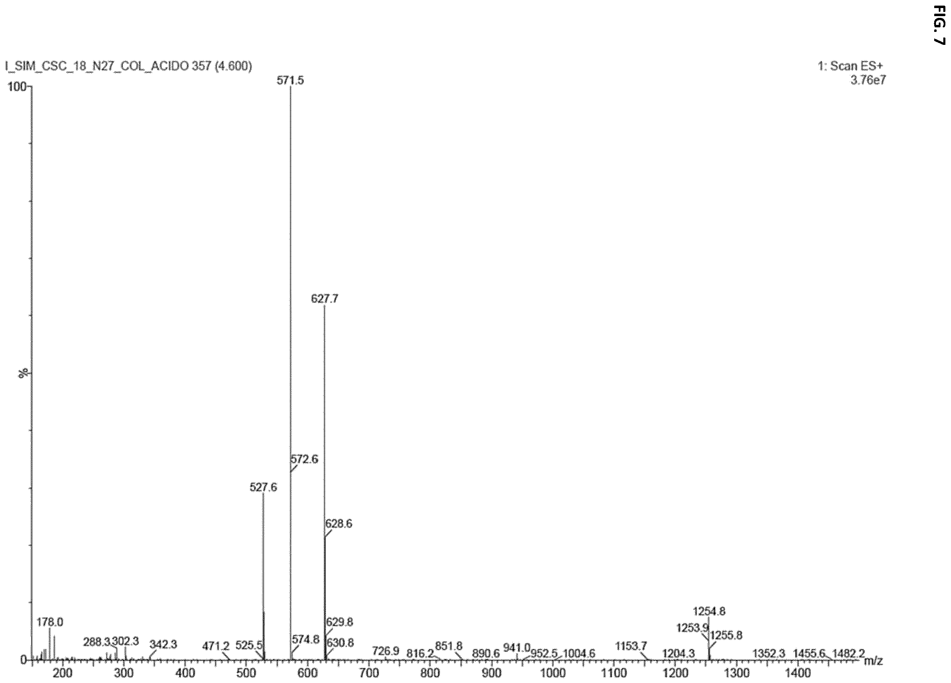

**Figure 7****.** Electrospray mass-spectrum of (2S,2'S)-di-tert-butyl 2,2'-(1,1'-([1,1'-biphenyl]-4,4'-diyl)bis(1H-1,2,3-triazole-4,1-diyl))bis(pyrrolidine-1-carboxylate).

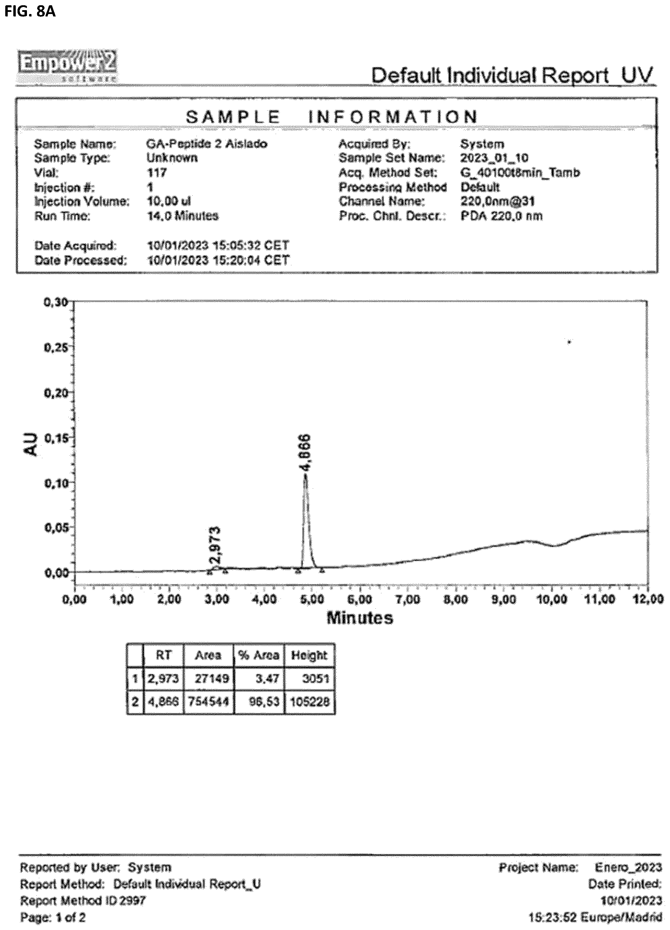

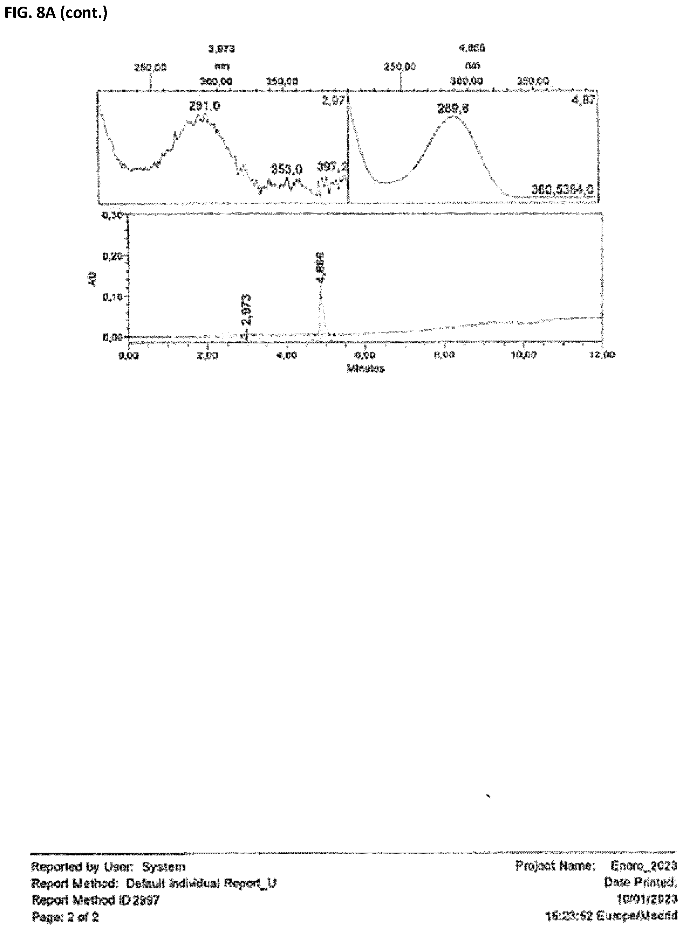

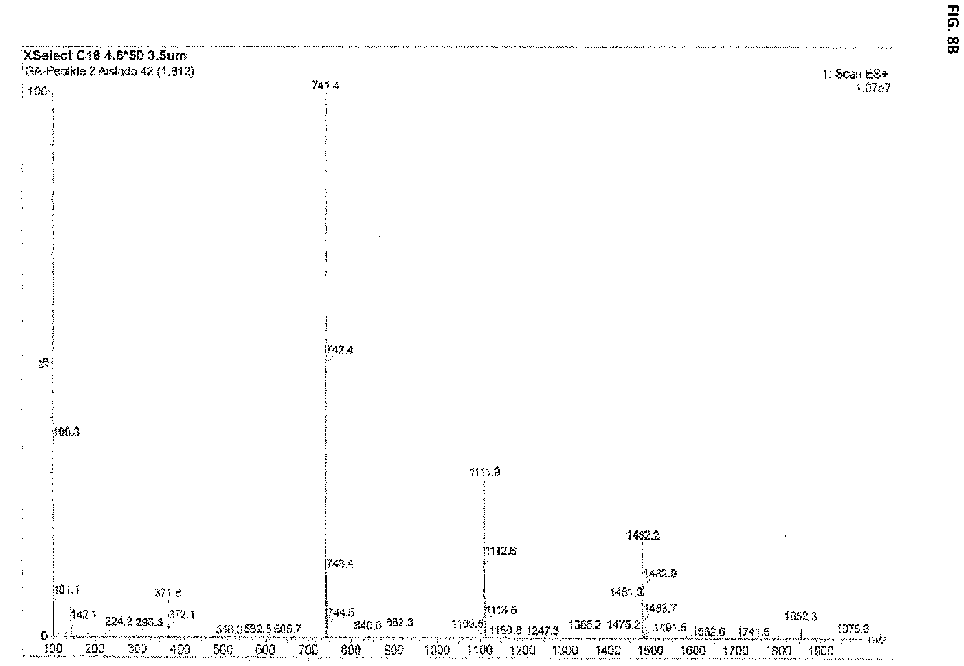

**Figure 8****.** A. HPLC characterization of compound 7 (V-Me). B. Electrospray mass-spectrum of compound 7 (V-Me).

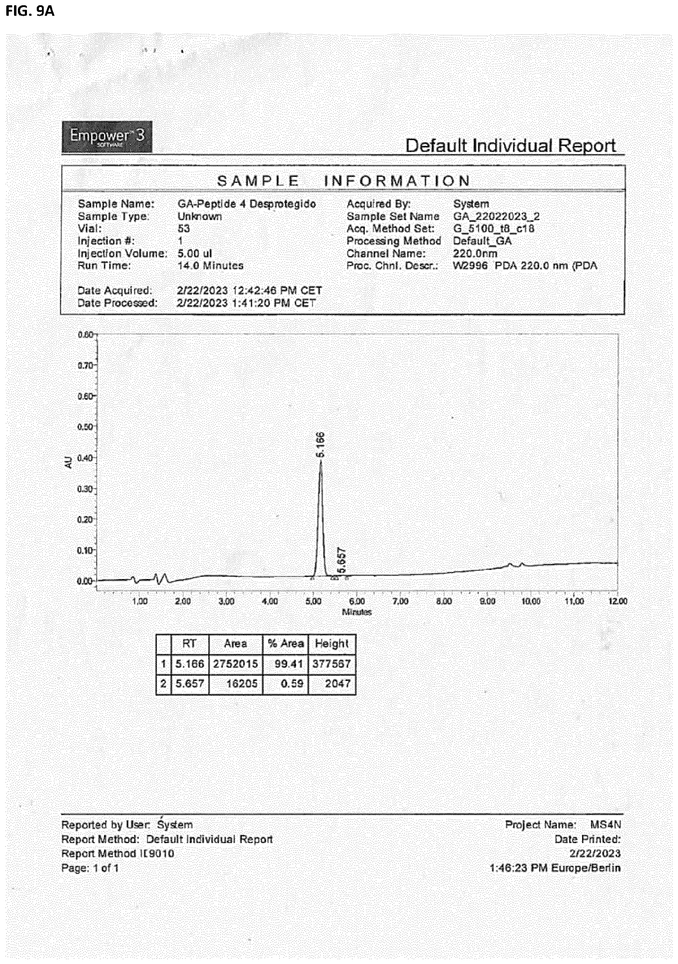

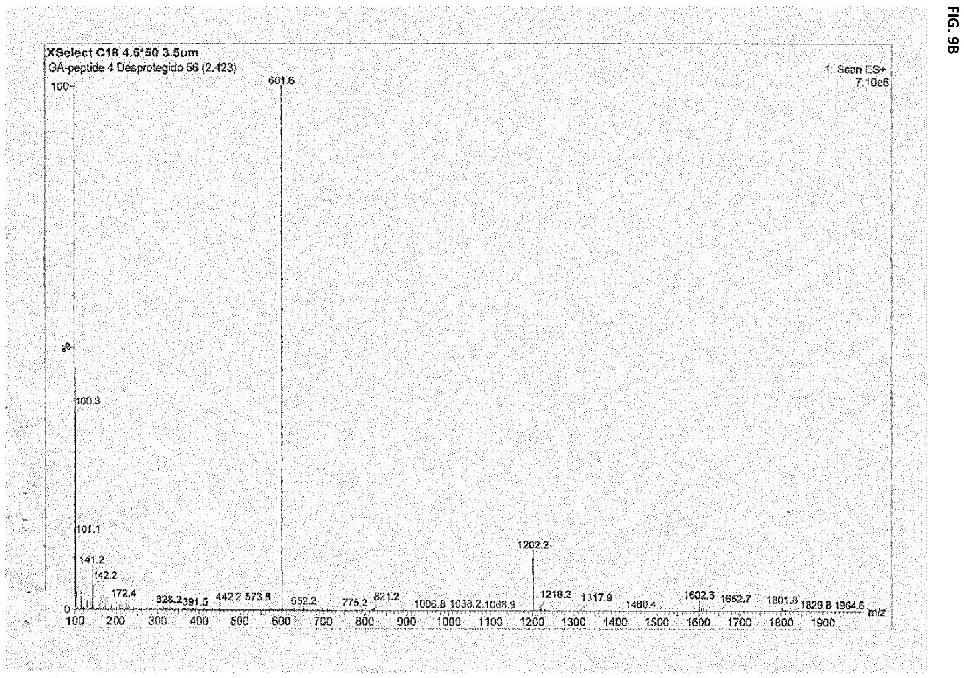

**Figure 9****.** A. HPLC characterization of compound 13 (SLE-Me). B. Electrospray mass-spectrum of compound 13 (SLE-Me).

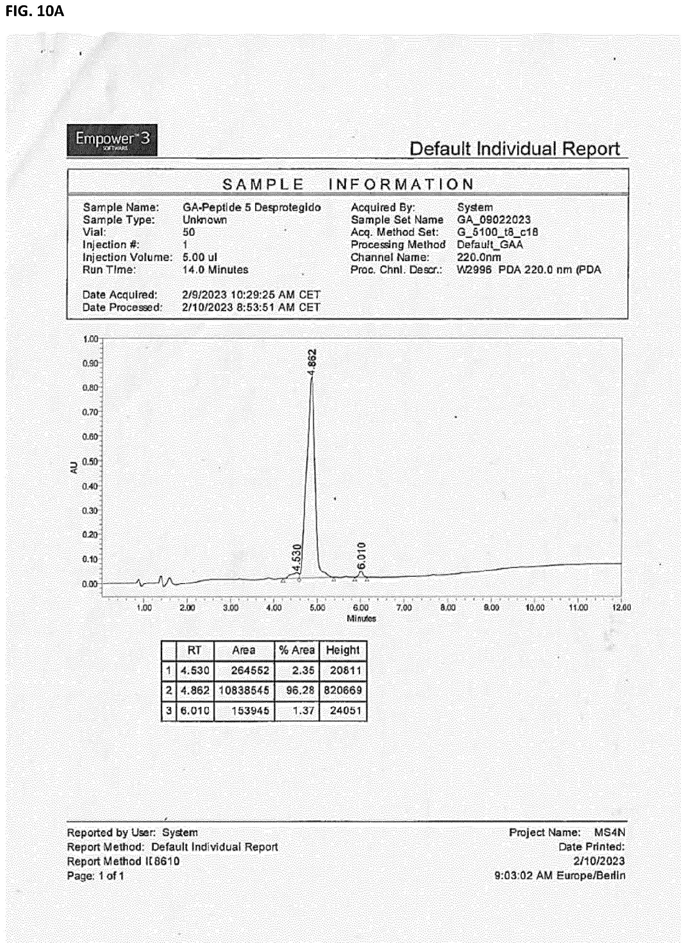

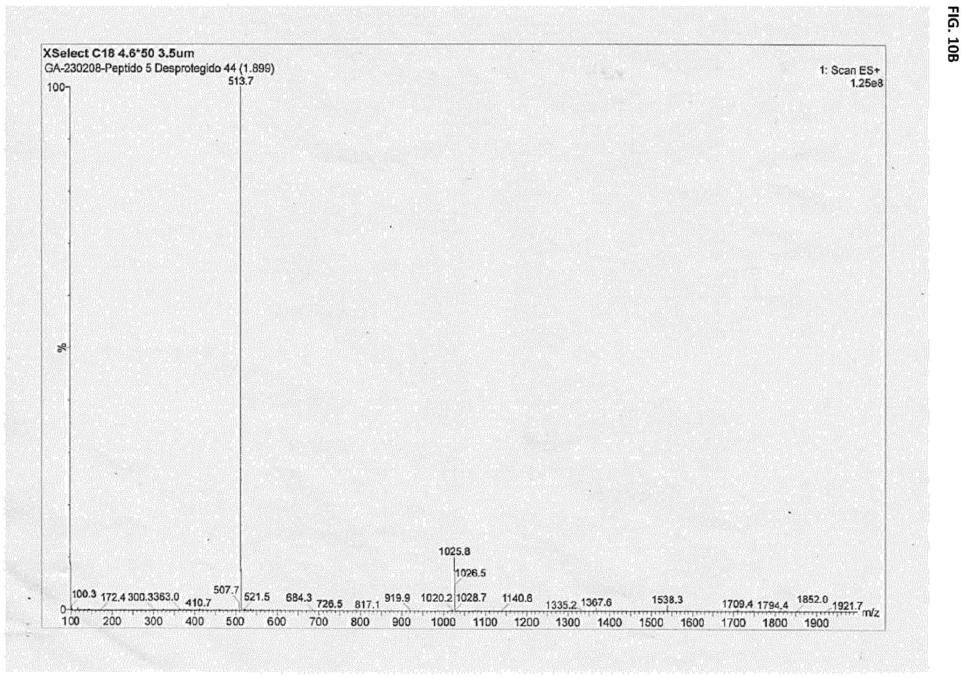

**Figure 10****.** A. HPLC characterization of compound 11 (LK-Me). B Electrospray mass-spectrum of compound 11 (LK-Me).

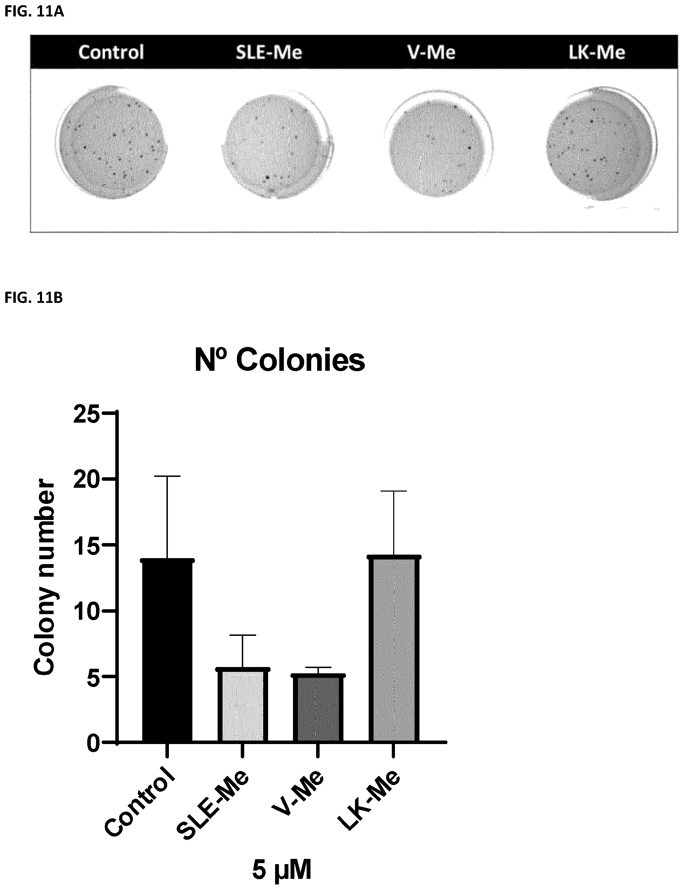

**Figure 11****.** Effect of drugs on colony formation.

Drugs SLE-Me and V-Me significantly decrease colony formation of Src transformed SW620 colorectal cancer cells, while a negative control compound (LK-Me) with the same hydrophobic scaffold but a different peptide sequence has no effect.

Cells were seeded on 24-well plate and cultured until reach 100% confluence. A. Representative photograph of colony formation. B. Statistical analysis of colony formation. Data is representative of two independent experiments. Data represent mean ± SEM (n=4/group in each experiment). Data were analyzed by ordinary one-way ANOVA followed by Dunnett's pos-hoc.

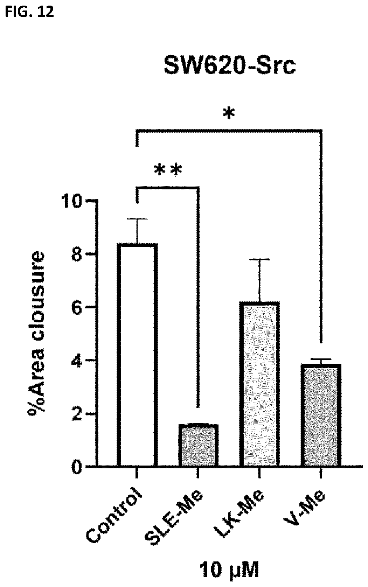

**Figure 12****.** Effect of drugs on scratch wound healing.

Drugs SLE-Me and V-Me significantly impair the migration of Src transformed SW62 colorectal cancer cells next to the wound borders, while a negative control compound (LK-Me) with the same hydrophobic scaffold but a different peptide sequence has no effect. Cells were seeded on 24-well plate and cultured until reach 100% confluence. Data represent mean ± SEM (n=4/group in each experiment). Data were analyzed by ordinary one-way ANOVA followed by Dunnett's pos-hoc.

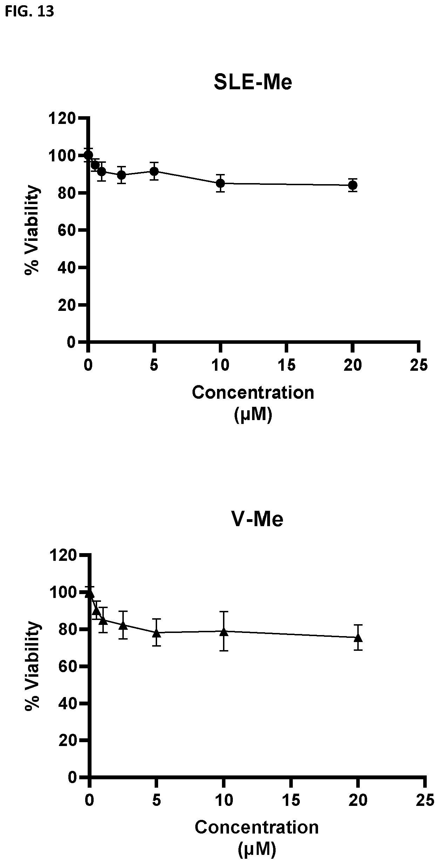

**Figure 13****.** Effect of drugs on cell viability.

Src transformed SW620 cells show no significant decrease in cell viability even at 20 mM concentration. Cells were seeded and treated with increasing dose of each drug and cell viability were evaluated using MTT assay. Data represents mean ± SEM (n=4-6/group in each experiment). Data were analyzed by two-way ANOVA followed by Dunnett's pos-hoc to compare with non-treated group.

### DETAILED DESCRIPTION OF THE INVENTION

### Definitions

Terms not specifically defined herein should be given the meanings that would be given to them by one of skill in the art in light of the disclosure and the context. As used in the specification, however, unless specified to the contrary, the following terms have the meaning indicated and the following conventions are adhered to.

Throughout the present specification and the accompanying clauses, the words "comprise" and variations such as "comprises", "comprising" are to be interpreted inclusively. That is, these words are intended to convey the possible inclusion of other elements or integers not specifically recited, where the context allows. The word "comprise" also includes the term "consists of".

In the context of the present invention, the term "salt" must be understood as any form of a compound used in accordance with this invention in which said compound is in ionic form or is charged and coupled to a counter-ion (a cation or anion) or is in solution. This definition also includes quaternary ammonium salts and complexes of the active molecule with other molecules and ions, particularly, complexes formed via ionic interactions. The definition includes in particular physiologically acceptable salts; this term must be understood as equivalent to "pharmacologically acceptable salts" or "pharmaceutically acceptable salts".

The term "isomer" refers to molecules of identical atomic compositions, but with different bonding arrangements of atoms or orientations of their atoms in space. Constitutional isomers are also named structural or positional isomers and are molecules with the same exact number of atoms but they differ in the bonding arrangements. Stereoisomers or spatial isomers are molecules with the same atomic composition and atom connectivity but different orientation of atoms in the space. Enantiomers are mirror images of each other that are non-superposable. Diastereomers are stereoisomers that are not mirror images of each other. Conformers have the same structural formula but different shapes that arise from rotations about one or more bonds. Atropisomers are stereoisomers which result from hindered rotation about a single bond, where the steric strain barrier to rotation is high enough to allow for the isolation of the conformers.

In the context of the present invention, the term "pharmaceutically acceptable salt" means any salt that is tolerated physiologically (normally meaning that it is not toxic, particularly, as a result of the counter-ion) when used in an appropriate manner for a treatment, applied or used, particularly, in humans and/or mammals. These physiologically acceptable salts may be formed with cations or bases and, in the context of this invention, are understood to be salts formed by at least one compound used in accordance with the invention -normally an acid (deprotonated)- such as an anion and at least one physiologically tolerated cation, preferably inorganic, particularly when used in humans and/or mammals. Salts with alkali and alkali earth metals are preferred particularly, as well as those formed with ammonium cations (NH₄⁺). Preferred salts are those formed with (mono) or (di)sodium, (mono) or (di)potassium, magnesium or calcium. These physiologically acceptable salts may also be formed with anions or acids and, in the context of this invention, are understood as being salts formed by at least one compound used in accordance with the invention - normally protonated, for example in nitrogen - such as a cation and at least one physiologically tolerated anion, particularly when used on humans and/or mammals. This definition specifically includes in the context of this invention a salt formed by a physiologically tolerated acid, i.e., salts of a specific active compound with physiologically tolerated organic or inorganic acids - particularly when used on humans and/or mammals. Examples of this type of salts are those formed with: hydrochloric acid, hydrobromic acid, sulphuric acid, methanesulfonic acid, formic acid, acetic acid, oxalic acid, succinic acid, malic acid, tartaric acid, mandelic acid, fumaric acid, lactic acid or citric acid.

The term "treatment" as used herein refers to the prophylactic and/or therapeutic treatment.

The term "therapeutic treatment" as used herein refers to bringing a body from a pathological state or disease back to its normal, healthy state. Specifically, unless otherwise indicated, includes the amelioration, cure, and/or maintenance of a cure (i.e., the prevention or delay of relapse) of a disease or disorder. Treatment after a disorder has started aims to reduce, alleviate, ameliorate or altogether eliminate the disorder, and/or its associated symptoms, to prevent it from becoming worse, to slow the rate of progression, or to prevent the disorder from re-occurring once it has been initially eliminated (i.e., to prevent a relapse). It is noted that, this term as used herein is not understood to include the term "prophylactic treatment" as defined herein.

The term "prophylactic treatment" or "preventive treatment" as used herein refers to preventing a pathological state. It is noted that, this term as used herein is not understood to include the term "therapeutic treatment" as defined herein.

The term "therapeutically effective amount" as used herein refers to an amount that is effective, upon single or multiple dose administration to a subject (such as a human patient) in the prophylactic or therapeutic treatment of a disease, disorder or pathological condition.

The term "subject" as used herein refers to a mammalian subject. Preferably, it is selected from a human, companion animal, non-domestic livestock or zoo animal. For example, the subject may be selected from a human, mouse, rat, dog, cat, cow, pig, sheep, horse, bear, and so on. In a preferred embodiment, said mammalian subject is a human subject.

The term "active ingredient" as used herein refers to any component that provides pharmacological activity.

The terms "agent" and "drug" are used herein interchangeably.

The term "medicament" describes a drug or combination of drugs that have the ability to treat or prevent diseases, disorders or conditions in subjects or that they can be used in subjects with the aim of restoring, correcting or modifying the physiological functions performing a pharmacological, immunological or metabolic function.

The terms "medicament" and "medication" are used herein interchangeably.

### Detailed description

The first aspect of the invention relates to a compound of formula (I): 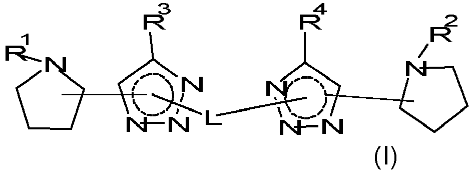
a pharmaceutically salt or isomer thereof,
wherein
R¹ and R² are the same or different and are selected from the group consisting of: a non-polar amino acid and a peptide, wherein
   - the non-polar amino acid or peptide:
      ∘ is N-terminal protected,
      ∘ binds to the N atom of the pyrrolidinyl ring by its C-terminal end, forming an amide bond, and
      ∘ the lateral chain of the one or more amino acid(s) is optionally protected.
   - the peptide comprises sequence SEQ ID NO: 1 (MGSNKSKPKDASQRRRSLEPAENVHGAGGG) or any fragment thereof;
R³ and R⁴ are the same or different and represent hydrogen, (C₁-C₅)alkyl, (C₂-C₅)alkenyl, (C₂-Cs)alkynyl, (C₁-C₅)alkyl substituted by one or more substituents Su₄, (C₂-C₅)alkenyl substituted by one or more substituents Su₅, or (C₂-C₅)alkynyl substituted by one or more substituents Su₆; and
L is of formula (II): 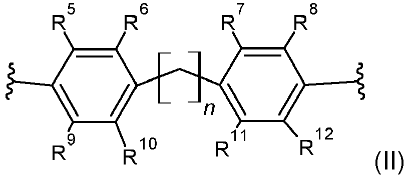
wherein
n is an entire number selected from 0 to 3, wherein when n=O, both rings are linked by a single bond;
R⁵ to R¹² are the same or different and are independently selected from the group consisting of: H, halogen, (C₁-C₁₀)alkyl, NO₂, OR¹³, SR¹⁴, NR¹⁵R¹⁶, and (+)NR¹⁷R¹⁸R¹⁹; or, alternatively,
R⁵ and R⁸ to R¹² are as defined above, and R⁶ and R⁷, together with the carbon atoms to which they are attached, form a ring system; or, alternatively,
R⁵ to R⁹ and R¹² are as defined above, and R¹⁰ and R¹¹ form, together with the carbon atoms to which they are attached, a ring system; or, alternatively,
R⁵, R⁸, R⁹ and R¹² are as defined above, R⁶ and R⁷ together with the carbon atoms to which they are attached form a ring system, and R¹⁰ and R¹¹ together with the carbon atoms to which they are attached form another ring system;
wherein the term "ring system" means a ring having from 6 to 10 members selected from CRₓ, C(RₓR_{y})₂, O, S, N, or NR_{y};
R¹³ and R¹⁴ represent hydrogen, (C₁-C₁₀)alkyl or (C₁-C₁₀) alkyl substituted by one or more substituents Su₇;
R¹⁵ to R¹⁹ are the same or different and are independently selected from the group consisting of: H, (C₁-C₁₀)alkyl, and (C₁-C₁₀)alkyl substituted by one or more substituents Su₈;
Rₓ and R_{y} represent hydrogen, (C₁-C₁₀)alkyl or (C₁-C₁₀) alkyl substituted by one or more substituents Su₉; and
Su₁ to Su₉ are independently selected from the group consisting of: halogen, cyano, nitro, (C₁-C₆)haloalkyl, (C₁-C₆)alkyl, (C₁-C₆)hydroxyalkyl, and (C₁-C₆)alkoxy.

The term (C₁-C₁₀)alkyl refers to a saturated straight or branched alkyl chain having from 1 to 10 carbon atoms. Illustrative non-limitative examples are: methyl, ethyl, propyl, isopropyl, butyl, isobutyl, sec-butyl, tert-butyl, n-pentyl, neo-pentyl and n-hexyl.

The term (C₁-C₅)alkyl refers to a saturated straight or branched alkyl chain having from 1 to 5 carbon atoms.

The term (C₂-C₅)alkenyl refers to a saturated straight, or branched alkyl chain containing from 2 to 5 carbon atoms and also containing one or more double bonds. Illustrative non-limitative examples are ethenyl, propenyl, butenyl, 1-methyl-2-buten-1-yl, and the like.

The term (C₂-C₅)alkynyl refers to a saturated straight, or branched alkyl chain containing from 2 to 5 carbon atoms and also containing one or more triple bonds. Examples include, among others, ethynyl, 1-propynyl, 2-butynyl, 1,3-butadinyl and 4-pentynyl.

The term (C₁-C₆)haloalkyl refers to a group resulting from the replacement of one or more hydrogen atoms from a (C₁-C₆)alkyl group with one or more, preferably from 1 to 6, halogen atoms, which can be the same or different. Examples include, among others, trifluoromethyl, fluoromethyl, 1-chloroethyl, 2-chloroethyl, 1-fluoroethyl, 2-fluoroethyl, 2-bromoethyl, 2-iodoethyl, 2,2,2-trifluoroethyl, pentafluoroethyl, 3-fluoropropyl, 3-chloropropyl, 2,2,3,3-tetrafluoropropyl, 2,2,3,3,3-pentafluoropropyl, heptafluoropropyl, 4-fluorobutyl, and nonafluorobutyl.

The term "halogen" refers to the group in the periodic table consisting of five chemically related elements: fluorine (F), chlorine (CI), bromine (Br), iodine (I), and astatine (At).

The term (C₁-C₆)alkoxy refers to a group resulting from the replacement of one or more hydrogen atoms from a (C₁-C₆)alkyl group with one or more, preferably from 1 to 6, oxygen atoms, which can be the same or different.

In the present invention, the term "amino acid" refers to a molecule containing both an amino group and a carboxyl group. Amino acids can be classified by the side chain group. There are basically four different classes of amino acids determined by different side chains: (1) non-polar, (2) polar and neutral, (3) acidic and polar, (4) basic and polar.

Non-polar amino acids have side chains which are hydrocarbon alkyl groups (alkane branches) or aromatic (benzene rings) or heteroaromatic (e.g. indole ring). Illustrative non-limitative examples of common non-polar amino acids are Ala, Val, Leu, Ile, Pro, Trp, Gly, Phe, and Met. Polar-neutral amino acids have polar but not charged groups at neutral pH in the side chain (such as hydroxyl, amide or thiol groups). Illustrative non-limitative examples of polar neutral amino acids are Ser, Thr, Cys, Tyr, Asn, and Gln.

Acid amino acids (hereinafter also referred as "acid and polar amino acid") have acidic side chains at neutral pH. These are aspartic acid or aspartate (Asp) and glutamic acid or glutamate (Glu), among others. Their side chains have carboxylic acid groups whose pKa's are low enough to lose protons, becoming negatively charged in the process. Basic amino acids (hereinafter also referred as "basic and polar amino acid") have side chains containing nitrogen and resemble ammonia which is a base (such as amines, guanidines, or imidazole). Their pKa's are high enough that they tend to bind protons, gaining a positive charge in the process. Illustrative non-limitative examples of basic amino acids are Lys, Arg, and His.

Suitable amino acids include, without limitation, alpha amino acids, such as the L-isomers of alpha-amino acids of the 20 common naturally occurring alpha-amino acids: alanine, arginine, asparagine, aspartic acid, cysteine, glutamine, glutamic acid, glycine, histidine, isoleucine, leucine, lysine, methionine, phenylalanine, proline, serine, threonine, tryptophan, tyrosine, and valine; natural beta-amino acids (e.g., beta-alanine); and unnatural amino acids.

The term "unnatural amino acid" comprises D-isomers of the 20 common naturally occurring alpha-amino acids. Further illustrative non-limitative examples of unnatural amino acids are summarized in Table I:

**Table I**

| | |
|---|---|
| Aad | 2-Aminoadipic acid |
| bAad | 3-Aminoadipic acid |
| bAla | beta-Alanine, beta-Aminopropionic acid |
| Abu | 2-Aminobutyric acid |
| 4Abu | 4-Aminobutyric acid, piperidinic acid |
| Acp | 6-Aminocaproic acid |
| Ahe | 2-Aminoheptanoic acid |
| Aib | 2-Aminoisobutyric acid |
| bAib | 3-Aminoisobutyric acid |
| Apm | 2-Aminopimelic acid |
| Dbu | 2,4 Diaminobutyric acid |
| Des | Desmosine |
| Dpm | 2,2'-Diaminopimelic acid |
| Dpr | 2,3-Diaminopropionic acid |
| EtGly | N-Ethylglycine |
| EtAsn | N-Ethylasparagine |
| Hyl | Hydroxylysine |
| aHyl | allo-Hydroxylysine |
| 3Hyp | 3-Hydroxyproline |
| 4Hyp | 4-Hydroxyproline |
| Ide | Isodesmosine |
| alle | allo-Isoleucine |
| Nva | Norvaline |
| Nle | Norleucine |
| Orn | Ornithine |

Each one of the amino acids forming the peptide of the invention can have, independently from the others, L- or D-configuration.

The N-protecting group can be any amino protecting group known in the art. Among the classes of amino protecting groups contemplated are: (1) acyl type protecting groups such as formyl, trifluoroacetyl, phthalyl, p-toluenesulfonyl (tosyl), benzenesulfonyl, nitrophenylsulfonyl, tritylsulfonyl, o-nitrophenoxyacetyl, and α-chlorobutryl; (2) aromatic urethane type protecting groups such as benzyloxycarbonyl and substituted benzyloxycarbonyls such as p-chlorobenzyloxycarbonyl, p-methoxybenzyloxycarbonyl, p-nitrobenzyloxycarbonyl, p-bromobenzyloxycarbonyl, 1-(p-biphenylyl)-1-methylethoxycarbonyl, α,α-dimethyl-3,5-dimethoxybenzyloxycarbonyl, and benzhydryloxycarbonyl; (3) aliphatic urethane protecting groups such as t-butyloxycarbonyl (Boc), diisopropylmethoxycarbonyl, isopropyloxycarbonyl, ethoxycarbonyl, and allyloxycarbonyl; (4) cycloalkyl urethane type protecting groups such as cyclopentyloxycarbonyl,adamantyloxycarbonyl,andcyclohexyloxycarbonyl; (5) thiourethane type protecting groups such as phenylthiocarbonyl; (6) alkyl type protecting groups such as triphenylmethyl (trityl) and benzyl (Bn); (7) trialkylsilane protecting groups such as trimethylsilane, 4-[-(4-chlorophenyl) sulfonylaminocarbonyl] phenyl carbonyl, and 4-[-(4-bromophenyl) sulfonylaminocarbonyl] phenyl carbonyl. The preferred α-amino protecting group is t-butyloxycarbonyl (Boc), methylurethane or ethylurethane; its use as an α-amino protecting group for amino acids is well known to those of skill in the art ([see, e.g., by Bodansky et al. in "The Practice of Peptide Synthesis," Springer-Verlag, Berlin (1984), p.20].

In the context of the invention, the term "peptide" refers to polymers from at least 2 amino acids, formed by a condensation reaction, joining together through a peptide bond. Sequential peptide bonds with additional amino acids yield a peptide chain and the building block of proteins. Amino acids being part of a peptide are also called "residues".

It is noted that the fragments of SEQ ID NO: 1 are defined as described below. In particular, the fragments of SEQ ID NO: 1 can comprise from 2 to 9 contiguous amino acids of SEQ ID NO: 1.

In one embodiment, R¹ and R² are the same or different and represent peptides as defined above; particularly, R¹ and R² are the same or different and comprise sequence SEQ ID NO: 1 MGSNKSKPKDASQRRRSLEPAENVHGAGGG or any fragment thereof. In a preferred embodiment, R¹ and R² are the same or different and comprise at least 2 contiguous amino acids contained in sequence SEQ ID NO: 1. In another preferred embodiment, R¹ and R² are the same or different and comprise at least 3 contiguous amino acids contained in sequence SEQ ID NO: 1. In another embodiment, R¹ and R² are the same or different and comprise at least 4 contiguous amino acids contained in sequence SEQ ID NO: 1. In another embodiment, R¹ and R² are the same or different and comprise at least 5 contiguous amino acids contained in sequence SEQ ID NO: 1. In another embodiment, R¹ and R² are the same or different and comprise at least 6 contiguous amino acids contained in sequence SEQ ID NO: 1. In another embodiment, R¹ and R² are the same or different and comprise at least 7 contiguous amino acids contained in sequence SEQ ID NO: 1. In another embodiment, R¹ and R² are the same or different and comprise at least 8 contiguous amino acids contained in sequence SEQ ID NO: 1. In another embodiment, R¹ and R² are the same or different and comprise at least 9 contiguous amino acids contained in sequence SEQ ID NO: 1.

In a more preferred embodiment, R¹ and R² are the same or different and comprise a fragment corresponding to SerLeuGlu contained in sequence SEQ ID NO: 1.

Alternatively to the compound of formula (I), in the present invention, the compound might also correspond to formula (Ib): 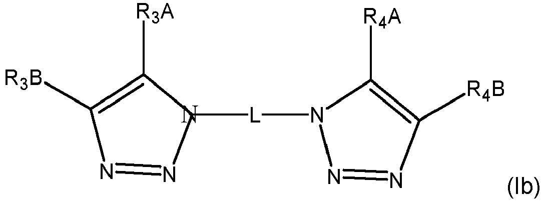 wherein
R₃A or R₃B is 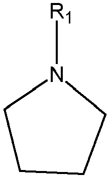 and
R₄A or R₄B is 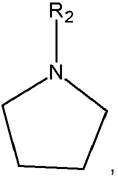
wherein one of R₃A or R₃B and one of R₄A or R₄B must be the above pyrrolidine ring substituted at the nitrogen with groups R₁ or R₂ that can be the same or different selected from the group consisting of a non-polar amino acid and a peptide as defined in the first aspect of the invention and the other (the R₃A or R₃B and the R₄A or R₄B not corresponding to the pyrrolidine ring) is as defined in R₃ or R₄ in the first aspect of the invention; and wherein the pyrrolidine ring binds to formula (lb) by the carbon of the pyrrolidine ring adjacent to the nitrogen atom.

In one embodiment of the first aspect of the invention, the compound corresponds to formula (Ic) or (Id): 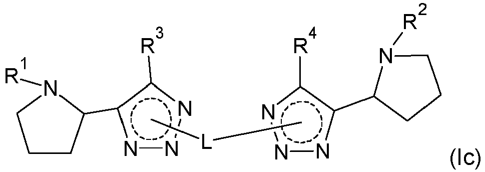 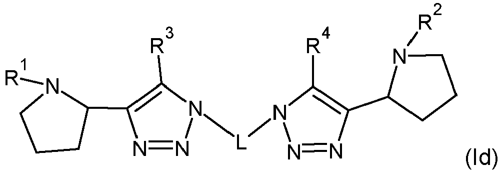 a pharmaceutically acceptable salt or isomer thereof, wherein R¹, R², R³, R⁴, and L are as defined in the first aspect.

In a preferred embodiment, the R¹ and R² of any of the previous compounds are the same or different and represent non-polar amino acids; particularly, wherein R¹ and R² are the same and represent non-polar amino acids. In another embodiment, R¹ and R² are different and represent non-polar amino acids.

In another embodiment, the R¹ and R² of compounds of formula (I), (Ib), (Ic) and (Id) are the same or different and represent peptides as defined above; particularly, R¹ and R² are the same or different and comprise a fragment corresponding to at least 2, 3, 4 or 5 contiguous amino acids contained in sequence SEQ ID NO: 1.

In a preferred embodiment, R¹ and R² are the same or different and comprise the fragment SerLeuGlu; particularly, R¹ and R² are the same and consist of amino acid sequence SerLeuGlu.

In another embodiment, R¹ and R² are the same or different and comprise Val; particularly, R¹ and R² are the same and consist of the amino acid Val.

In working embodiments, R¹ and R² are the same or different and comprise Val and/or the fragment SerLeuGlu.

In a non-working embodiment, R¹ and R² are the same or different and comprise the fragment LeuLys.

In a preferred embodiment, R¹ and R² comprise the same N-protecting group and it is one including an acyl (C=O) or ester group (-OCO-), such as acetyl, benzoyl, pivaloyl, tert-butyloxycarbonyl (BOC), N-Allyloxycarbonyl (Alloc), Benzyl chloroformate (Cbz), methylurethane or ethylurethane; particularly the N-protecting group is methylurethane or BOC.

In another preferred embodiment, R³ and R⁴ are the same, particularly hydrogen. In another embodiment, R³ and R⁴ are different.

In another preferred embodiment, R⁵ to R¹² are the same or different and are independently selected from the group consisting of H, halogen, (C₁-C₁₀)alkyl, NO₂, OR¹³, SR¹⁴, NR¹⁵R¹⁶, and (+)NR¹⁷R¹⁸R¹⁹; particularly R⁵ to R¹² are hydrogen.

In another preferred embodiment, n is zero.

In another preferred embodiment, the compound is of formula (le): 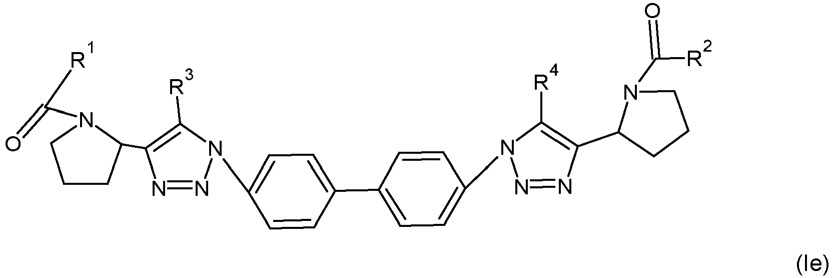 or a pharmaceutically acceptable salt or isomer thereof, wherein R¹, R², R³ and R⁴ are as previously defined.

In a preferred embodiment, in the compound of formula (le), R¹ and R² are the same and correspond to the amino acid peptide SerLeuGlu, wherein the side chains of Ser and Glu residues are optionally blocked. In another embodiment, R¹ and R² are the same and correspond to the amino acid Val.

In a preferred embodiment, in the compound of formula (le), R¹ and R² are N-terminal protected with the same group, which is selected from methyl urethane or Boc.

In a preferred embodiment, in the compound of formula (le), R³ and R⁴ are the same, particularly they represent hydrogen.

In a more preferred embodiment, the compound of the invention is the compound of formula (If):

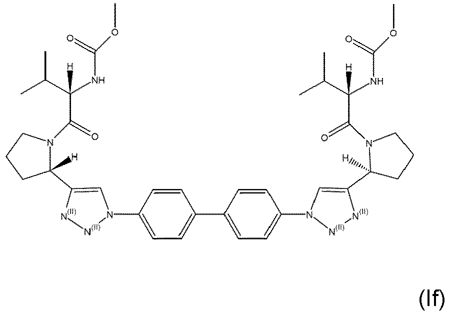

In a more preferred embodiment, the compound of the invention is the compound of formula (Ig):

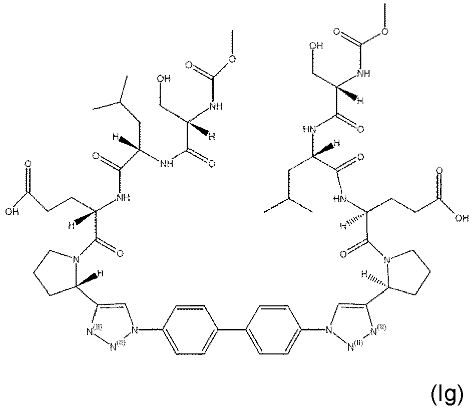

In a second aspect, the present invention also provides a pharmaceutical composition comprising a therapeutically effective amount of the compound as defined in any of the preceding embodiments together with one or more pharmaceutically acceptable excipients and/or carriers.

Pharmaceutically acceptable excipients include, but are not limited to a carrier or diluent, such as a gum, a starch (e.g. corn starch, pregeletanized starch), a sugar (e.g. lactose, mannitol, sucrose, dextrose), a cellulosic material (e.g. microcrystaline cellulose), an acrylate (e.g. polymethylacrylate), calcium carbonate, magnesium oxide, talc, or mixtures thereof; a binder (e.g. acacia, cornstarch, gelatin, carbomer, ethyl cellulose, guar gum, hydroxypropyl cellulose, hydroxypropyl methyl cellulose, povidone); a disintegrating agent (e.g. cornstarch, potato starch, alginic acid, silicon dioxide, croscarmelose sodium, crospovidone, guar gum, sodium starch glycolate), a buffer (e.g. Tris-HCl, acetate, phosphate, bicarbonate) of various pH and ionic strength; and additive such as albumin or gelatin to prevent absorption to surfaces; a detergent (e.g. Tween 20, Tween 80, Pluronic F68, bile acid salts); a protease inhibitor; a surfactant (e.g. sodium lauryl sulfate); a permeation enhancer; a solubilizing agent (e.g. glycerol, polyethylene glycerol); an antioxidants (e.g. ascorbic acid, sodium metabisulfite, butylated hydroxyanisole); a stabilizer (e.g. hydroxypropyl cellulose, hydroxypropylmethyl cellulose); a viscosity increasing agent (e.g. carbomer, colloidal silicon dioxide, ethyl cellulose, guar gum); a sweetener (e.g. aspartame, citric acid); a preservative (e.g. thimerosal, benzyl alcohol, parabens); a lubricant (e.g. stearic acid, magnesium stearate, polyethylene glycol, sodium lauryl sulfate); a flow-aid (e.g. colloidal silicon dioxide), a plasticizer (e.g. diethyl phthalate, triethyl citrate); an emulsifier (e.g. carbomer, hydroxypropyl cellulose, sodium lauryl sulfate); a polymer coating (e.g. poloxamers or poioxamines); a coating and film forming agent (e.g. ethyl cellulose, acrylates, polymethacrylates); a pharmaceutically acceptable carrier for liquid formulations, such as an aqueous (water, alcoholic/aqueous solution, emulsion or suspension, including saline and buffered media) or non-aqueous (e.g., propylene glycol, polyethylene glycol, and injectable organic esters, such as ethyl oleate) solution, suspension, emulsion or oil; and a parenteral vehicle (e.g., for subcutaneous, intravenous, intraarterial, or intramuscular injection), including but not limited to, water, oils, saline solution, Ringer's dextrose, aqueous dextrose and other sugar solutions. A pharmaceutically acceptable excipient also includes excipients for nanoencapsulation purposes, such as a cationic polyelectrolyte (e.g. gelatin and an anionic polyelectrolyte (e.g. arabic gum).

In some embodiments, said composition is a solid composition, preferably a stable solid composition. As used herein, a "stable composition" may refer to a formulation in which the active ingredient therein essentially retains its physical stability and/or chemical stability and/or biological activity upon storage.

Examples of polymer stabilizers suitable in various embodiments include, but are not limited to, polyelectrolytes such as, e.g., poly(acrylic acid), poly (styrene sulfonate), poly(diallyldimethylammonium chloride), poly(allylamine hydrochloride) (PAH), or others. Suitable examples of adsorbates include similar polyelectrolytes. In various preferred embodiments employing an adsorbate, the polymer stabilizer is of a larger molecular weight than the adsorbate moieties. In various embodiments, a preferred cationic group is the amino group and preferred anionic groups are carboxylic acid, sulfonic acid, phosphates, and the like. For cationic polymers, examples include, but are not limited to, poly(allylamine), poly(ethyleneimine), poly(diallyldimethylammonium chloride), poly(arginine), chitosan, cationic collapsible proteins, poly(methacrylamido propyl trimethyl ammonium chloride) and poly(lysine). For anionic polymers, examples include, but are not limited to, poly(acrylic acid), poly(styrene sulfonic acid), poly(glutamic acid), poly(methacrylic acid), poly(aspartic acid), nucleic acids, anionic collapsible proteins, poly (anetholesulfonic acid), cellulose, poly(maleic acid) poly(vinyl phosphoric acid), etc. Block polymers are made up of blocks of polymers having different functional groups. The block polymers can be made up of blocks of any of the mentioned anionic and cationic polymers and another polymer that imparts a specific desirable property to the block polymer.

It is contemplated that any embodiment discussed in this specification can be implemented with respect to any medical use, pharmaceutical composition, method of treatment, method of manufacturing a medicament and combination therapies of the invention, and vice versa. It will be understood that particular embodiments described herein are shown by way of illustration and not as limitations of the invention. The principal features of this invention can be employed in various embodiments without departing from the scope of the invention. Those skilled in the art will recognize, or be able to ascertain using no more than routine experimentation, numerous equivalents to the specific procedures described herein. Such equivalents are considered to be within the scope of this invention and are covered by the claims.

The third aspect of the invention relates to a compound described in any of the preceding embodiments for use in therapy.

The fourth aspect of the invention relates to a compound described in any of the preceding embodiments for use in the treatment of cancer, wherein there is an activation of Src in said cancer.

In addition, the present invention provides a method of treating cancer, wherein there is an activation of Src in said cancer, comprising administering to a subject in need of such treatment a prophylactic or therapeutically effective amount of a compound described in any of the preceding embodiments.

It further provides the use of a compound described in any of the preceding embodiments in the manufacture of a medicament for the preventive or therapeutic treatment of cancer, wherein there is an activation of Src in said cancer.

The term "cancer" describes a wide range of diseases, which share a common feature of uncontrolled cell growth, leading to the formation of tumors in any part, organ or tissue of the body. Tumors may promote the formation of new blood vessels, in a process called angiogenesis. These abnormal blood vessels may be used for tumor cells to migrate from its tissue or organ of origin and reach other organs. This process is called metastasis or tumor dissemination. The terms "cancer", "tumor", "neoplasm" and "neoplasia" are used herein interchangeably.

Cancer types comprise acute lymphoblastic leukemia (ALL), acute myeloid leukemia (AML), adrenocortical carcinoma, AIDS-related cancers such as Kaposi sarcoma (soft tissue sarcoma), AIDS-related lymphoma (lymphoma), primary CNS lymphoma (lymphoma); anal cancer, astrocytomas, atypical teratoid/rhabdoid tumor, basal cell carcinoma of the skin, carcinoma of unknown primary, central nervous system (including atypical teratoid/rhabdoid tumor, medulloblastoma and other CNS embryonal tumors, germ cell tumor, primary CNS lymphoma), cervical cancer, childhood cancers, childhood cardiac tumors treatment, childhood Rare Cancers, cholangiocarcinoma, chordoma, chronic lymphocytic leukemia (CLL), chronic myelogenous leukemia (CML), chronic myeloproliferative neoplasms, colorectal cancer, craniopharyngioma, cutaneous T-cell lymphoma, ductal carcinoma in situ (DCIS), embryonal tumors, medulloblastoma and other central nervous system cancers, endometrial cancer (uterine cancer), ependymoma, esophageal cancer, esthesioneuroblastoma (head and neck cancer), Ewing sarcoma (bone cancer), extracranial germ cell tumor, extragonadal germ cell tumor, eye cancer (intraocular melanoma and retinoblastoma), fallopian tube cancer, gallbladder cancer, gastric (stomach) cancer, gastrointestinal neuroendocrine tumors, gastrointestinal stromal tumors (GIST) (soft tissue sarcoma), germ cell tumors (including childhood central nervous system germ cell tumors (brain cancer), childhood extracranial germ cell tumors, extragonadal germ cell tumors, ovarian germ cell tumors and testicular cancer), gestational trophoblastic disease, hairy cell leukemia, head and neck cancer, heart tumors, hepatocellular (liver) cancer, Hodgkin lymphoma, hypopharyngeal cancer (head and neck cancer), intraocular melanoma, islet cell tumors (pancreatic neuroendocrine tumors), Kaposi sarcoma (soft tissue sarcoma), kidney (renal cell) cancer, Langerhans cell histiocytosis, laryngeal cancer (head and neck cancer), leukemia, lip and oral cavity cancer (head and neck cancer), liver cancer, lung cancer (non-small cell, small cell, pleuropulmonary blastoma, pulmonary inflammatory myofibroblastic tumor, and tracheobronchial tumor), lymphoma, male breast cancer, melanoma, intraocular melanoma (eye), Merkel cell carcinoma (skin cancer), malignant mesothelioma, metastatic cancer, metastatic squamous neck cancer with occult primary (head and neck cancer), midline tract carcinoma with NUT gene changes, mouth cancer (head and neck cancer), multiple endocrine neoplasia syndromes, multiple myeloma/plasma cell neoplasms, mycosis fungoides (lymphoma), myelodysplastic syndromes, myelodysplastic/myeloproliferative neoplasms, chronic myelogenous leukemia (CML), acute myeloid leukemia (AML), chronic myeloproliferative neoplasms, nasal cavity and paranasal sinus cancer (head and neck cancer), nasopharyngeal cancer (head and neck cancer), neuroblastoma, neuroendocrine tumors (gastrointestinal), non-Hodgkin lymphoma, non-small cell lung cancer, oral cancer, lip and oral cavity cancer and oropharyngeal cancer (head and neck cancer), osteosarcoma and undifferentiated pleomorphic sarcoma of bone treatment, ovarian cancer, pancreatic cancer, pancreatic neuroendocrine tumors (islet cell tumors), papillomatosis (childhood laryngeal), paraganglioma, paranasal sinus and nasal cavity cancer (head and neck cancer), parathyroid cancer, penile cancer, pharyngeal cancer (head and neck cancer), pheochromocytoma, pituitary tumor, pleuropulmonary blastoma (lung cancer), primary central nervous system (CNS) lymphoma, primary peritoneal cancer, prostate cancer, pulmonary inflammatory myofibroblastic tumor (lung cancer), rectal cancer, recurrent cancer, retinoblastoma, childhood rhabdomyosarcoma (soft tissue sarcoma), salivary gland cancer (head and neck cancer), sarcoma, childhood vascular tumors (soft tissue sarcoma), Sézary syndrome (lymphoma), skin cancer, small cell lung cancer, small intestine cancer, soft tissue sarcoma squamous cell carcinoma of the skin, metastatic squamous neck cancer with occult primary, (head and neck cancer), testicular cancer, throat cancer (head and neck cancer), nasopharyngeal cancer, oropharyngeal cancer, hypopharyngeal cancer, thymoma and thymic carcinoma, thyroid cancer, tracheobronchial tumors (lung cancer), transitional cell cancer of the renal pelvis and ureter (kidney (renal cell) cancer), carcinoma of unknown primary, ureter and renal pelvis, transitional cell cancer (kidney (renal cell) cancer, urethral cancer, uterine sarcoma, vaginal cancer, vascular tumors (soft tissue sarcoma), vulvar cancer and Wilms tumor and other childhood kidney tumors (National Cancer Institute. (21June 2023). Cancer Types. https://www.cancer.gov/types).

Human proto-oncogene tyrosine-protein kinase Src, also named c-Src or pp60^{c-Src}, is a 60 kD protein with intrinsic tyrosine kinase activity encoded by SRC gene. It is the homologue to transforming v-Src viral oncogene. c-Src mediates signal transduction through the interaction with multiple proteins and protein complexes in the cytoplasm. The Src family of kinases (SFK) are a group of non-receptor kinases that include Src, Fun, Yes, Yrk, Fgr, Hck, Blk, Lck and Lyn. Src comprises a unique domain at the N-terminal region, a negative tyrosine regulatory region (Tyr530), and four Src homology (SH) domains, SH4, SH3, SH2 and the kinase domain SH1 (Summy JM, Gallick GE. Src family kinases in tumor progression and metastasis. Cancer Metastasis Rev. 2003;22(4):337-358. doi:10.1023/a:1023772912750). Src is activated by autophosphorylation at Tyr419 of the SH1 domain, while the interaction between the SH2 domain and phosphorylated Tyr530 results in Src inactivation (Yeatman TJ. A renaissance for SRC. Nat Rev Cancer. 2004;4(6):470-480. doi:10.1038/nrc1366; Kim LC, Song L, Haura EB. Src kinases as therapeutic targets for cancer. Nat Rev Clin Oncol. 2009;6(10):587-595. doi:10.1038/nrclinonc.2009.129; Yaffe MB. Phosphotyrosine-binding domains in signal transduction. Nat Rev Mol Cell Biol. 2002;3(3):177-186. doi:10.1038/nrm759)

c-Src activation has been reported in more than 50% of tumors arising from colon, liver, lung, breast and pancreas (Dehm SM, Bonham K. SRC gene expression in human cancer: the role of transcriptional activation. Biochem Cell Biol. 2004;82(2):263-274. doi:10.1139/o03-077). In particular, increased activity and/or the expression of c-Src has been found in colon tumors. Around 80% of colon carcinomas have shown an enhanced tyrosine kinase activity of c-Src compared to normal colon mucosa (Cartwright CA, Meisler Al, Eckhart W. Activation of the pp60c-src protein kinase is an early event in colonic carcinogenesis. Proc Natl Acad Sci U S A. 1990;87(2):558-562. doi:10.1073/pnas.87.2.558). In colorectal carcinoma metastasis, an enhanced activity of c-Src has been identified in both hepatic and extrahepatic metastasis in comparison with normal colonic epithelia and primary colon tumors (Talamonti MS, Roh MS, Curley SA, Gallick GE. Increase in activity and level of pp60c-src in progressive stages of human colorectal cancer. J Clin Invest. 1993;91(1):53-60. doi:10.1172/JCI116200). Increased c-Src activity has been reported as an indicator of poor clinical prognosis in all stages of colon cancer (Aligayer H, Boyd DD, Heiss MM, Abdalla EK, Curley SA, Gallick GE. Activation of Src kinase in primary colorectal carcinoma: an indicator of poor clinical prognosis. Cancer. 2002;94(2):344-351. doi:10.1002/cncr.10221).

The mechanisms that lead to an activation of c-Src in human cancers have been studied. An activating SRC mutation, Q531 stop, has been described in 12% of highly advanced of colon cancers (Irby RB, Mao W, Coppola D, et al. Activating SRC mutation in a subset of advanced human colon cancers. Nat Genet. 1999;21(2):187-190. doi:10.1038/5971). However, this mutation may be considered a rare event. Other works have reported that the activation of c-Src in colon cancer is associated to an increase of its specific activity (Cartwright CA, Kamps MP, Meisler Al, Pipas JM, Eckhart W. pp60c-src activation in human colon carcinoma. J Clin Invest. 1989;83(6):2025-2033. doi:10.1172/JCI114113), although not a clear associated between one of the activating c-Src kinases, Csk, an increase in c-Src activation has been established yet. The increase in c-Src protein expression is human cancers is one of the most consistent findings that explain c-Src enhanced activity (Biscardi JS, Tice DA, Parsons SJ. c-Src, receptor tyrosine kinases, and human cancer. Adv Cancer Res. 1999;76:61-119. doi:10.1016/s0065-230x(08)60774-5).

In a preferred embodiment of the fourth aspect, the cancer is colorectal cancer.

In another embodiment, the colorectal cancer has an activation of c-Src activity. In another embodiment, the colorectal cancer has a mutation, an increase of the specific activity and/or an increase of the expression of c-Src protein.

Apart from cancer, an increase in Src activity has been described to cause thrombocytopenia, myelofibrosis, bleeding, platelet dysfunction with abnormal α-granules, and bone pathologies (Turro E, Greene D, Wijgaerts A, et al. A dominant gain-of-function mutation in universal tyrosine kinase SRC causes thrombocytopenia, myelofibrosis, bleeding, and bone pathologies. Sci Transl Med. 2016;8(328):328ra30. doi:10.1126/scitranslmed.aad7666).

All publications and patent applications are herein incorporated by reference to the same extent as if each individual publication or patent application was specifically and individually indicated to be incorporated by reference.

The use of the word "a" or "an" may mean "one," but it is also consistent with the meaning of "one or more," "at least one," and "one or more than one". The use of the term "another" may also refer to one or more. The use of the term "or" in the claims is used to mean "and/or" unless explicitly indicated to refer to alternatives only or the alternatives are mutually exclusive.

As used in this specification and claim(s), the words "comprising" (and any form of comprising, such as "comprise" and "comprises"), "having" (and any form of having, such as "have" and "has"), "including" (and any form of including, such as "includes" and "include") or "containing" (and any form of containing, such as "contains" and "contain") are inclusive or open-ended and do not exclude additional, unrecited elements or method steps. The term "comprises" also encompasses and expressly discloses the terms "consists of" and "consists essentially of". As used herein, the phrase "consisting essentially of" limits the scope of a claim to the specified materials or steps and those that do not materially affect the basic and novel characteristic(s) of the claimed invention. As used herein, the phrase "consisting of excludes any element, step, or ingredient not specified in the claim except for, e.g., impurities ordinarily associated with the element or limitation.

The term "or combinations thereof' as used herein refers to all permutations and combinations of the listed items preceding the term. For example, "A, B, C, or combinations thereof" is intended to include at least one of: A, B, C, AB, AC, BC, or ABC, and if order is important in a particular context, also BA, CA, CB, CBA, BCA, ACB, BAC, or CAB. Continuing with this example, expressly included are combinations that contain repeats of one or more item or term, such as BB, AAA, AB, BBC, AAABCCCC, CBBAAA, CABABB, and so forth. The skilled artisan will understand that typically there is no limit on the number of items or terms in any combination, unless otherwise apparent from the context.

As used herein, words of approximation such as, without limitation, "about", "around", "approximately" refers to a condition that when so modified is understood to not necessarily be absolute or perfect but would be considered close enough to those of ordinary skill in the art to warrant designating the condition as being present. The extent to which the description may vary will depend on how great a change can be instituted and still have one of ordinary skilled in the art recognize the modified feature as still having the required characteristics and capabilities of the unmodified feature. In general, but subject to the preceding discussion, a numerical value herein that is modified by a word of approximation such as "about" may vary from the stated value by ±1, 2, 3, 4, 5, 6, 7, 8, 9, 10, 11, 12, 13, 14 or 15%. Preferably the term "about" means exactly the indicated value (± 0%).

The following examples serve to illustrate the present invention and should not be construed as limiting the scope thereof.

### EXAMPLES

### Example 1. - Synthetic procedures of the hydrophobic moiety

### Materials and methods

All the Chemicals and solvents are from commercial suppliers and used without purification, except the anhydrous solvents such as DMF which were treated previously through a system of solvent purification (PureSolv), degasified with inert gases and dried over alumina or molecular sieves. Reactions were monitored by thin layer chromatography (60 F, 0.2 mm, Macherey-Nagel) by visualisation under 254 and/or 365 nm lamp. Purification was made by Flash column chromatography by using Merck Silica Gel 60, 40-63 microns RE. NMR were performed in a Brucker 400 MHz. HPLC 2795 Alliance Waters Aquity coupled to Detector DAD Agilent 1100 and Detector MS Waters ESI triple quadrupole Quattro micro, 10 µL of sample in ACN was injected, using a ZORBAX Extend-C18 3.5 µm 2.1x50mm (Agilent) column. The mobile phase used was a mixture of A = water + 0.05 formic acid and B = ACN + 0.05 formic acid with method described as follows: flow 0.5 mL/min; 5% B for 0.5 min; 5% to 100% B in 5 min, 100% B for 2min. Mass spectroscopy (MS) analysed by FIA with coupled to LCT Premier Orthogonal Accelerated Time of Flight Mass Spectrometer, acquiring data by ESI in positive mode. Spectra have been scanned between 50 and 1500 Da with values every 0.2 seconds and peaks are given m/z (% of basis peak).

### Synthetic procedures

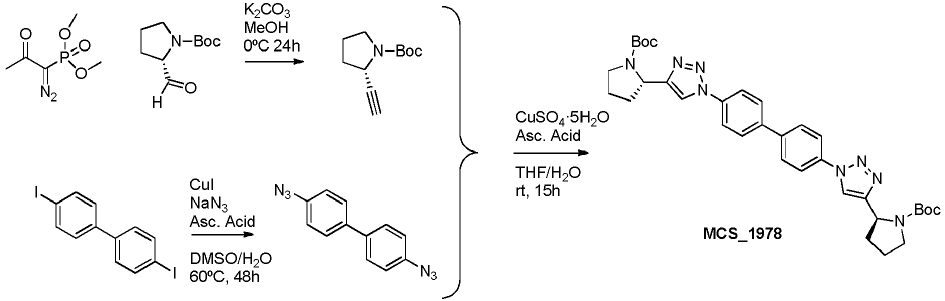

***(S)*-*tert*-butyl 2-ethynylpyrrolidine-1-carboxylate:** To a suspension of (*S*)-*tert*-butyl 2-formylpyrrolidine-1-carboxylate (500 µL, 2,67 mmol) and K₂CO₃ (737 mg, 5,34 mmol) in 13 mL of Methanol at 0°C, dimethyl (1-diazo-2-oxopropyl)phosphonate (520 µL, 3,47 mmol) was added dropwise. After stirring for 24 h, the mixture was diluted with EtOAc and water, the organic layer was washed with brine, dried over with Na₂SO₄, filtered and concentrated in vacuum. The residue was purified by flash chromatography (silica gel, Hex:EtOAc 2: 1) to afford 329 mg desiderate compound (63%).¹H-NMR was identical to the previously described in the literature (Polymer Chemistry, 11 (18), 2020, 3179-3187). **4,4'-diazido-1,1'-biphenyl:** To a mixture of ,4'-diiodo-1,1'-biphenyl (1 g, 2,463 mmol), copper(I) iodate (0,141 g, 0,739 mmol), sodium azide (0,400 g, 6,16 mmol) and sodium 2-(1,2-dihydroxyethyl)-4-hydroxy-5-oxo-2,5-dihydrofuran-3-olate (0,146 g, 0,739 mmol), a mixture of 22mL of DMSO and 2 mL of water was added. The reaction mixture was stirred 48h at 60°C then quenched with water/ EtOAc and washed with brine (x3). The organic layer was dried over with Na2SO4, filtered and concentrated in vacuum. The residue was purified by flash chromatography (silica gel, Hex:EtOAc 4:1) to afford 100 mg of desiderate compound (17%). ¹H-NMR was identical to the previously described in the literature (Journal of Material Chemistry, 22(21), 2012, 10472-10479).

**(2S,2'S)-di-tert-butyl 2,2'-(1,1'-([1,1'-biphenyl]-4,4'-diyl)bis(1H-1,2,3-triazole-4,1-diyl))bis(pyrrolidine-1-carboxylate) (MCS-1978):** To a mixture of (S)-tert-butyl 2-ethynylpyrrolidine-1-carboxylate (150mg, 0,768 mmol), 4,4'-diazido-1,1'-biphenyl (70 mg, 0,296 mmol) , sodium 2-(1,2-dihydroxyethyl)-4-hydroxy-5-oxo-2,5-dihydrofuran-3-olate (29,4 mg, 0,148 mmol) and copper(II) sulfate, 5H2O (18,50 mg, 0,074 mmol), a mixture of 3mL of THF and 0.6 mL of water was added. The reaction mixture was stirred 15h at rt then quenched with water/ EtOAc and washed with brine. The organic layer was dried over with Na₂SO₄, filtered and concentrated in vacuum. The residue was purified by flash chromatography (silica gel, Hex:EtOAc 1:4) to afford 145 mg of desiderate compound (78%). HPLC-Ms rt= 4.50 min; m/z: 627.7 [M+1]⁺.

### Example 2. - Synthetic procedures of the peptides

### Material and Methods

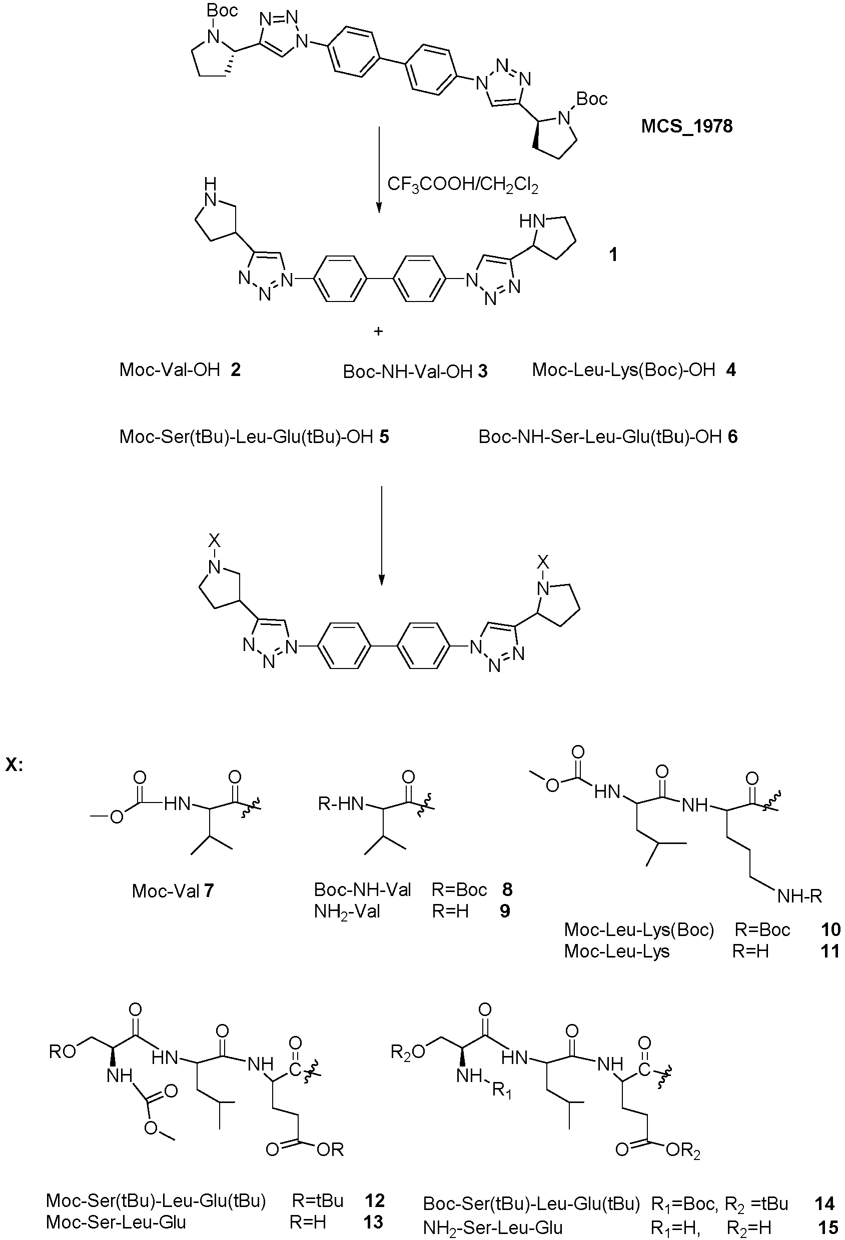

### Synthesis of 1-(4'-(4-(pyrrolidin-2-yl)-1H-1,2,3-triazol-1-yl)-[1,1'-biphenyl]-4-yl)-4-(pyrrolidin-3-yl)-1l4,2,3l2-triazole intermediate (1)

The compound MCS_1978 was dissolved in CH₂Cl₂ and CF₃COOH was added until a ratio of CF₃COOH:CH₂Cl₂ (4:6, v/v) was obtained and the evolution of the reaction was monitored by HPLC. At the end of the reaction, the solvent was evaporated under vacuum and the crude compound was dissolved in a solution of CH₃CN:H₂O (7:3, v/v) and lyophilised. The final compound (1) was used for further reactions without purification. HPLC: X Bridge BEH C₁₈ reversed-phase analytical column (4.6 mm X 50 mm, 5µm), linear gradient 5-100% CH₃CN (0.035% CF₃COOH) in H₂O (0.045% CF₃COOH) over 8 min. at a flow rate of ¹mL/min, tr: 4.295 min., 99% (at 220nm). LR-MS: calculated mass for C₃₄H₄₂N₈O₄ 423.2280 [M], 427.2314 [M+H]¹⁺, found by HPLC-MS: 427.7 [M+H]¹⁺, 214.2 [M+2H]²⁺.

### General method for the synthesis of the peptide moieties

### Peptide moieties synthesized:

Moc-Leu-Lys(Boc)-OH **(4)**
Moc-Ser(tBu)-Leu-Glu(tBu)-OH **(5)**
Boc-Ser(tBu)-Leu-Glu(tBu)-OH **(6)**

Peptide moieties were synthesized by a Fmoc/tBu solid phase strategy using 2-CI-Trt resin (1.6 mmol CI/g) as a solid support. The first amino acid (Fmoc-Lys(Boc)-OH for the peptide moiety **4** and Fmoc-Glu(tBu)-OH) and for the peptide moieties **5** and **6**) was incorporated onto the 2-Cl-Trt resin by addition of the corresponding Fmoc-amino acid (1mmol) and *N,N-*diisopropylethylamine (DIEA, 8mmol) dissolved in dimethylformamide (DMF) anhydride. After 1.5 h, CH₃OH was added and allowed to react for a 30 further min. The mixture was then filtered and washed with DMF and dichloromethane (DCM). The temporary Fmoc group was removed using a 20% piperidine solution in DMF with two treatments of 5 min each, followed by DMF washes. Peptide extension was performed by coupling the corresponding Fmoc amino acids to the amino acid resin for 1 h using diisopropylcarbodiimide (DIC) and Oxyma-K as coupling reagents (3 eq of each reagent) and DMF as solvent.

For dipeptide **4** the last amino acid was introduced with the α-amino protected with the methyloxycarbonyl (Moc) group. In the case of tripeptides **5** and **6,** once completion of the peptidyl moieties, Fmoc removal of the N-terminal amine and DMF and DCM washes, the NH₂-Ser(tBu)-Leu-Glu(tBu)-peptidyl resin was separated into two fractions. Both were modified at the N-terminal amine, one with the Moc group with methyl chloroformate (3 eq.) and DIEA (6 eq.) in DMF **(5)** and the other with the t-butyloxycarbonyl group (Boc) with Boc₂O and DIEA (2 eq. of each reagent) in DMF **(6).** Eight treatments with a solution of CF₃COOH: DCM (1:99, v/v) for 1 min released peptidyl moieties from the resin which were collected in water. The DCM was evaporated under vacuum and the resulting solid was dissolved in CH₃CN and lyophilized. The peptide moieties were used in further reactions without purification.

### General process for the incorporation of the protected amino acids or the peptidyl moieties into the 1-(4'-(4-(pyrrolidin-2-yl)-1H-1,2,3-triazol-1-yl)-[1,1'-biphenyl]-4-yl)-4-(pyrrolidin-3-yl)-1l4,2,3l2-triazole intermediate

The incorporation of protected the amino acids (Moc-Val-OH or Boc-Val-OH) and the peptidyl moieties **(4, 5** and **6)** into intermediate **1** (1-(4'-(4-(pyrrolidin-2-yl)-1H-1,2,3-triazol-1-yl)-[1,1'-biphenyl]-4-yl)-4-(pyrrolidin-3-yl)-1l4,2,3l2-triazole) was performed in solution using DMF as solvent and a mixture of the intermediate (1 eq.), the corresponding protected amino acid or protected peptide (2 eq.), EDC (2 eq.) and HOBt (2 eq.). The evolution of the reaction was monitored by HPLC. At the end of the reaction, DCM was added and the mixture was washed with a 5% solution of NaHCOs and sat. NH₄Cl solution, and finally dried with anh. Na₂SO₄. The solvent was evaporated under vacuum and the resulting solid was dissolved in a solution of CH₃CN:H₂O (7:3, v/v) and lyophilised.

Each lyophilised pellet was divided into two parts, one part was retained with all the protecting groups (final compounds **7, 8, 10, 12** and **14**) and the other part was treated with a solution of CF₃COOH:DCM (6:4, v/v) for 30 min to remove the protecting groups (final compounds **9, 11, 13** and **15**)**.** The solvent was then evaporated under vacuum and dissolved in CH₃CN:H₂O (1:1, v/v) and lyophilised. All final compounds were purified by semipreparative HPLC with a X Bridge Peptide BEH C18 OBD Prep column (130 Å, 5 µm, 19 x100 mm), using H₂O (1% CF₃COOH) and CH₃CN (1% CF₃COOH) as eluents. Final compounds were analyzed and characterized by HPLC and HPLC-MS.

Moc-Val-**1**-Val-Moc **(7):** 12.8 mg (white solid). HPLC: X Bridge BEH C₁₈ reversed-phase analytical column (4.6 mm X 50 mm, 5µm), linear gradient 40-100% CH₃CN (0.035% CF₃COOH) in H₂O (0.045% CF₃COOH) over 8 min. at a flow rate of 1mL/min, tr: 4.886 min., 96% (at 220nm). LR-MS: calculated mass for 740.3758 [M], 741.3792[M+H]¹⁺, found by HPLC-MS 741.4 [M+H]¹⁺, 371.5 [M+2H]²⁺.

Boc-Val-**1**-Val-Boc **(8):** 5.1 mg (white solid). HPLC: X Bridge BEH C₁₈ reversed-phase analytical column (4.6 mm X 50 mm, 5µm), linear gradient 40-100% CH₃CN (0.035% CF₃COOH) in H₂O (0.045% CF₃COOH) over 8 min. at a flow rate of 1mL/min, tr: 6.806 min., 91% (at 220nm). LR-MS: calculated mass for C₄₄H₆₀N₁₀O₆ 824.4697 [M], 825.4731 [M+H]¹⁺, found by HPLC-MS 825.6 [M+H]¹⁺, 413.6 [M+2H]²⁺.

NH₂-Val-**1**-Val-NH₂ **(9):** 10.7 mg (white solid). HPLC: X Select C₁₈ reversed-phase analytical column (4.6 mm X 50 mm, 3.5 µm), linear gradient 10-70% CH₃CN (0.07% HCOOH) in H₂O (0.1% HCOOH) over 8 min. at a flow rate of 1mL/min, tr: 5.021 min., 90% (at 220nm). LR-MS: calculated mass for C₃₄H₄₄N₁₀O₂ 624.3649 [M], 625.3682 [M+H]¹⁺, found by HPLC-MS: 625.6 [M+H]¹⁺, 313.6 [M+2H]²⁺.

Moc-Leu-Lys(Boc)-1-Lys(Boc)-Leu-Moc **(10):** 5.5 mg (white solid). HPLC: X Bridge BEH C₁₈ reversed-phase analytical column (4.6 mm X 50 mm, 5µm), linear gradient 50-100% CH₃CN (0.035% CF₃COOH) in H₂O (0.045% CF₃COOH) over 8 min. at a flow rate of 1mL/min, tr: 7.493 min, 96% (at 220nm). LR-MS: calculated mass for C₆₂H₉₂N₁₄O₁₂ 1124.7019 [M], 1225.7053 [M+H]¹⁺, found by HPLC-MS: 1225.5 [M+H]¹⁺, 613.7 [M+2H]²⁺.

Moc-Leu-Lys-**1**-Lys-Leu-Moc **(11):** 15.9 mg (white solid). HPLC: X Bridge BEH C₁₈ reversed-phase analytical column (4.6 mm X 50 mm, 5µm), linear gradient 5-100% CH₃CN (0.035% CF₃COOH) in H₂O (0.045% CF₃COOH) over 8 min. at a flow rate of 1 mL/min, tr: 4.862 min, 96% (at 220nm). LR-MS: calculated mass for C₅₂H₇₆N₁₄O₈₁ 1024.5971 [M], 1025.6004 [M+H]¹⁺, found by HPLC-MS: 1025.8 [M+H]¹⁺, 513.7 [M+2H]²⁺.

Moc-Ser(tBu)-Leu-Glu(tBu)-**1**-Glu(tBu)-Leu-Ser(tBu)-Moc **(12):** 4.2 mg (white solid) . HPLC: X Bridge BEH C₁₈ reversed-phase analytical column (4.6 mm X 50 mm, 5µm), linear gradient 50-100% CH₃CN (0.035% CF₃COOH) in H₂O (0.045% CF₃COOH) over 8 min. at a flow rate of 1mL/min, tr: 6.318 min, 96% (at 220nm). LR-MS: calculated mass for C₇₂H₁₀₈N₁₄O₁₆ 1424.8068 [M], 1425.8101 [M+H]¹⁺, found by HPLC-MS: 1425.7 [M+H]¹⁺, 713.8 [M+2H]²⁺.

Moc-Ser-Leu-Glu-**1**-Glu-Leu-Ser-Moc **(13):** 9.0 mg (white solid). HPLC: X Bridge BEH C₁₈ reversed-phase analytical column (4.6 mm X 50 mm, 5µm), linear gradient 5-100% CH₃CN (0.035% CF₃COOH) in H₂O (0.045% CF₃COOH) over 8 min. at a flow rate of 1mL/min, tr: 5.166 min, 99% (at 220nm). LR-MS: calculated mass for C₅₆H₇₆N₁₄O₁₆ 1200.5564 [M], 1201.5597 [M+H]¹⁺, found by HPLC-MS: 1202.2 [M+H]¹⁺, 601.6 [M+2H]²⁺. Boc-Ser(tBu)-Leu-Glu(tBu)-1-Glu(tBu)-Leu-Ser(tBu)-Boc **(14):** 5.3 mg (white solid). HPLC: X Bridge BEH C₁₈ reversed-phase analytical column (4.6 mm X 50 mm, 5µm), linear gradient 50-100% CH₃CN (0.035% CF₃COOH) in H₂O (0.045% CF₃COOH) over 8 min. at a flow rate of 1mL/min, tr: 8.434 min, 95% (at 220nm). LR-MS: calculated mass for C₇₈H₁₂₀N₁₄O₁₆ 1508.007 [M], 1509.9040 [M+H]¹⁺, found by HPLC-MS: 1510.5 [M+H]¹⁺, 755.9 [M+2H]²⁺, 1518.8 [M+H+ H₂O]¹⁺, 764.3 [M+2H+H₂O]²⁺.

NH₂-Ser-Leu-Glu-1-Glu-Leu-Ser-NH₂ **(15):** 13 mg (white solid). HPLC: X Bridge BEH C₁₈ reversed-phase analytical column (4.6 mm X 50 mm, 5µm), linear gradient 5-100% CH₃CN (0.035% CF₃COOH) in H₂O (0.045% CF₃COOH) over 8 min. at a flow rate of 1mL/min, tr: 4.481 min, 99% (at 220nm). LR-MS: calculated mass for C₅₂H₇₂N₁₄O₁₂ 1084.5454 [M], 1085.5488 [M+H]¹⁺, found by HPLC-MS:1086.0 [M+H]¹⁺, 543.7 [M+2H]²⁺.

### 1-(4'-(4-(pyrrolidin-2-yl)-1H-1,2,3-triazol-1-yl)-[1,1'-biphenyl]-4-yl)-4-(pyrrolidin-3-yl)-1l4,2,3l2-triazole intermediate (1)

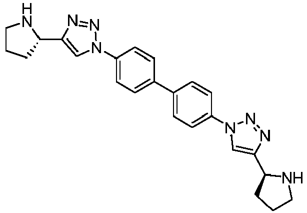

### Moc-Val-1-Val-Moc (7)

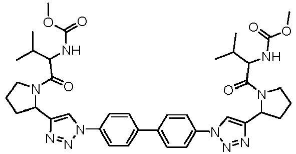

### Boc-Val-1-Val-Boc (8)

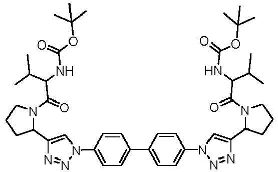

### NH₂-Val-1-Val-NH₂ (9)

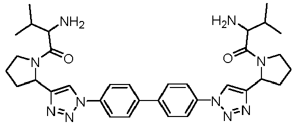

### Moc-Leu-Lys(Boc)-1-Lys(Boc)-Leu-Moc (10)

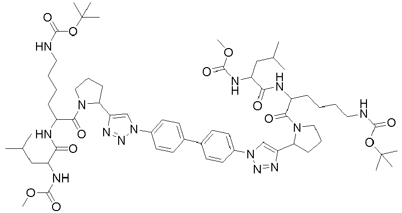

### Moc-Leu-Lys-1-Lys-Leu-Moc (11)

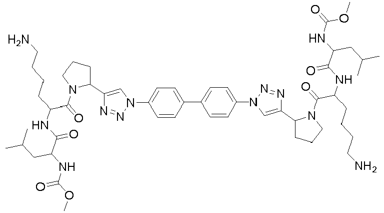

### Moc-Ser(tBu)-Leu-Glu(tBu)-1-Glu(tBu)-Leu-Ser(tBu)-Moc (12)

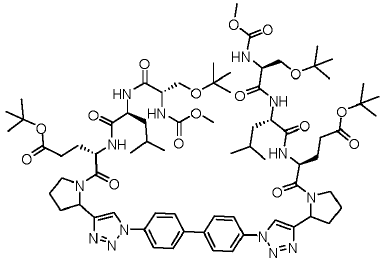

### Moc-Ser-Leu-Glu-1-Glu-Leu-Ser-Moc (13)

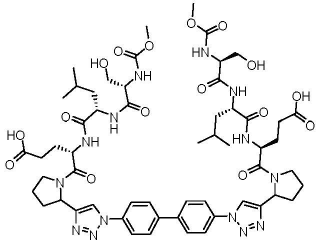

### Boc-Ser(tBu)-Leu-Glu(tBu)-1-Glu(tBu)-Leu-Ser(tBu)-Boc (14)

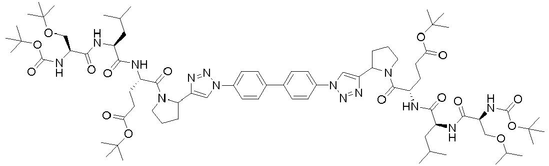

### NH₂-Ser-Leu-Glu-1-Glu-Leu-Ser-NH₂ (15)

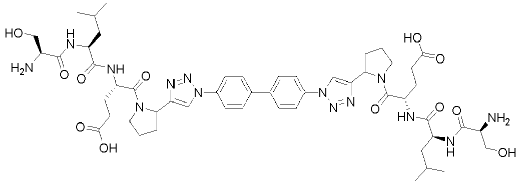

### Example 3. - Anticancer activity of the compounds of the invention

### Material and Methods

### Cell culture

SW620-src colorectal cancer cells were a kind gift of Dr. Serge Roche group. These cells have been stably transfected with human Src as described in Aponte, E., et al. (2021). "Regulation of Src tumor activity by its N-terminal intrinsically disordered region." Oncogene, 41: 960-970.. SW620-src colorectal cancer cells were grown in DMEM supplemented with 10% FBS (Gibco 10500-064), 1% GlutaMAX (Gibco 35050038) 1% P/S (Corning 30-002-CI). Cell lines were grown at 37°C with an atmosphere of 95% air, 5% CO2. All the reagents used during cell culture procedures were heated to 37°C in a water bath before its use. Before each assay, the absence of mycoplasma was tested according to a previously described protocol (Molla Kazemiha, V., et al. (2009). "PCR-based detection and eradication of mycoplasmal infections from various mammalian cell lines: a local experience." Cytotechnology 61(3): 117-124).

### Cell viability assay

Cell viability was measured by MTT assay as described before (Kamiloglu, S., et al. (2020). "Guidelines for cell viability assays." Food Frontiers 1(3): 332-349).. For this purpose, MTT solution was prepared by dissolving MTT in Dulbecco's phosphate-buffered saline (DPBS, Corning 21-031-CV) at 5 mg/ml. This solution is filtered and sterilized through a 0.2-µm filter into a sterile condition. MTT solution was stored at -20°C until analysis.

SW620-src cells were seeded into 96-well plates containing 100 µL of the growth medium at a density of 10000 cells/well for and cultured for one day. The following day, the medium was changed to a treatment medium in the absence or presence of varying concentrations of Drugs and incubated during 72h. After the incubation period, 10 µL of MTT solution was added to each well and an incubation for 2h was performed. Then, the medium was removed from the plates, and the MTT crystals were homogenized by adding 100 µL of acidified isopropyl alcohol (0.04 N HCl). To homogenize the pellets more efficiently, the plates were shaken for 5 min on a shaker. Afterwards, the plates were read under 570/690 nm wavelengths by plate reader (Synergy HTX, Biotek). Finally, cell viability was calculated subtracting 690 nm to 570 nm and dividing this result by non-treated cells.

### Soft agar colony formation assay

Cells were plated at density of 10,000 per well in 24-well plates in 0.3% soft agar on top of a base layer of 0.7% agar. The cells were treated with 5 µM of Drugs, and the plates were incubated for 3 weeks, replacing the media every week. At the end of incubation period, colonies were stained adding 200 µl of nitroblue tetrazolium chloride solution (1 mg/mL) per well and incubating plates overnight at 37°C. Once colonies are stained, photographs were taken with optical stereomicroscope (MZ216F, Leica), then we proceed to count colonies using imaged software (Schindelin, J., et al. (2012). "Fiji: an open-source platform for biological-image analysis." Nature Methods 9(7): 676-682).. These experiments were repeated twice with 4 replicates per group, and only colonies with >50 cells were counted.

### Wound healing assay

Cells were plated into 24-well plate at density of 500,000 cells/well and were cultured until they reach a 100% confluence. Then, the cells were washed with sterile PBS 1X and cell monolayer were cross-shape scratched with a 200-µL micropipette tip. Thereafter, cells were washed again with sterile PBS 1X twice, to remove detached cells and then 1 mL of DMEM supplemented with 1% FBS was added. Each well was photographed to record scratch distance and then incubated for 24h at 37°C in a humidified, 5% CO2 incubator. Each well was photographed in the same site to quantify cell migration after 24 hours. Every time, each well was photographed four times, one per each side of the cross, and the values obtained were averaged to obtain a representative value of each well. The photograph was taken using optical microscope (DMi8, Leica) with 5X objective. The obtained images were analyzed by Imaged software (Schindelin, J., et al. (2012). "Fiji: an open-source platform for biological-image analysis." Nature Methods 9(7): 676-682) using the MRI wound healing tool plugin (Suarez-Arnedo, A., et al. (2020). "An image J plugin for the high throughput image analysis of in vitro scratch wound healing assays." PLOS ONE 15(7): e0232565) . These experiments were repeated twice with 4 replicates per group.

### Statistical analysis

The statistical analyses performed indicate a comparison between controls and treatments of each experimental model. Ordinary one-way ANOVA was performed when more than two groups were compared, followed by Dunnet's post-hoc. Student's t test statistical analysis was used when two groups were compared. Statistically significant differences were considered when the level of confidence was above 95% (p-value < 0.05). Figures represent data expressed as mean ± standard error of mean (SEM). The number of samples per group used in each experiment is specified in each figure legend. All statistical analyses and figures have been generated using the GraphPad Prism 9.5.1 (GraphPad Software).

### Results

Essentially these experiments show that the SLE-Me and V-Me compounds have a significant effect on two properties that correlate with the aggressiveness of colorectal SW620 cells transformed by Src expression.

These transformed cells are highly aggressive and show rapid growth manifested by colony formation and a strong migratory capacity, which allows rapid colonisation of areas where cells have been killed. In the presence of the drugs SLE-Me and V-Me, both colony formation (Fig. 11 A and B) and migration of cancer cells are significantly reduced (Fig. 12). Both drugs show a low level of toxicity and do not significantly affect cell viability even at concentrations much higher than those used in biological activity assays (Fig. 13). The anticancer activity depends on the sequence of the peptides attached to the hydrophobic backbone. The compound LK-Me which has the same hydrophobic skeleton and the same terminal protecting group as SLE-Me or V-Me, but with a peptide sequence derived from another site of the Src protein, does not show significant biological activity against Src-activated SW620 cells in growth and migration assays.

### ITEMS OF THE INVENTION

**1.** A compound of formula (I): 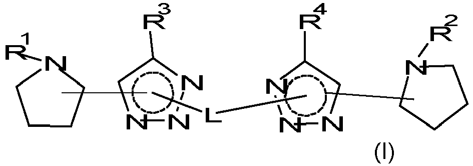
   a pharmaceutically salt or isomer thereof,
   wherein
   R¹ and R² are the same or different and are selected from the group consisting of: a non-polar amino acid and a peptide, wherein
      - the non-polar amino acid or peptide:
         ∘ is N-terminal protected,
         ∘ binds to the N atom of the pyrrolidinyl ring by its C-terminal end, forming an amide bond, and
         ∘ the lateral chain of the one or more amino acid(s) is optionally protected;
      - the peptide has a length from 2 to 9 amino acids, and comprises a fragment corresponding from 2 to 9 contiguous amino acids of sequence SEQ ID NO: 1(MGSNKSKPKDASQRRRSLEPAENVHGAGGG);
   R³ and R⁴ are the same or different and represent hydrogen, (C₁-C₅)alkyl, (C₂-C₅)alkenyl, (C₂-Cs)alkynyl, (C₁-C₅)alkyl substituted by one or more substituents Su₄, (C₂-C₅)alkenyl substituted by one or more substituents Su₅, or (C₂-C₅)alkynyl substituted by one or more substituents Su₆; and
   L is of formula (II): 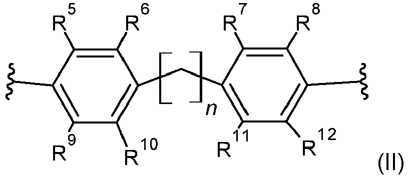
   wherein
   n is an entire number selected from 0 to 3, wherein when n=O, both rings are linked by a single bond;
   R⁵ to R¹² are the same or different and are independently selected from the group consisting of: H, halogen, (C₁-C₁₀)alkyl, NO₂, OR¹³, SR¹⁴, NR¹⁵R¹⁶, and (+)NR¹⁷R¹⁸R¹⁹; or, alternatively,
   R⁵ and R⁸ to R¹² are as defined above, and R⁶ and R⁷, together with the carbon atoms to which they are attached, form a ring system; or, alternatively,
   R⁵ to R⁹ and R¹² are as defined above, and R¹⁰ and R¹¹ form, together with the carbon atoms to which they are attached, a ring system; or, alternatively,
   R⁵, R⁸, R⁹ and R¹² are as defined above, R⁶ and R⁷ together with the carbon atoms to which they are attached form a ring system, and R¹⁰ and R¹¹ together with the carbon atoms to which they are attached form another ring system;
   wherein the term "ring system" means a ring having from 6 to 10 members selected from CRₓ, C(RₓR_{y})₂, O, S, N, or NR_{y};
   R¹³ and R¹⁴ represent hydrogen, (C₁-C₁₀)alkyl or (C₁-C₁₀) alkyl substituted by one or more substituents Su₇;
   R¹⁵ to R¹⁹ are the same or different and are independently selected from the group consisting of: H, (C₁-C₁₀)alkyl, and (C₁-C₁₀)alkyl substituted by one or more substituents Su₈;
   Rₓ and R_{y} represent hydrogen, (C₁-C₁₀)alkyl or (C₁-C₁₀) alkyl substituted by one or more substituents Su₉; and
   Su₁ to Su₉ are independently selected from the group consisting of: halogen, cyano, nitro, (C₁-C₆)haloalkyl, (C₁-C₆)alkyl, (C₁-C₆)hydroxyalkyl, and (C₁-C₆)alkoxy.
**2.** The compound as defined in item 1, which corresponds to formula (Ib) or (Ic): 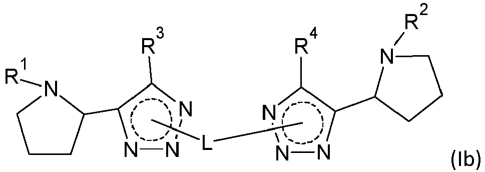 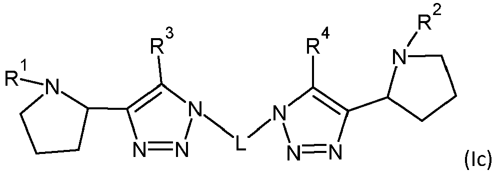 a pharmaceutically acceptable salt or isomer thereof, wherein R¹, R², R³, R⁴, and L are as defined in item 1.
**3.** The compound of any one of the preceding items, wherein R¹ and R² are the same or different and represent non-polar amino acids; particularly, wherein R¹ and R² are the same and represent non-polar amino acids.
**4.** The compound of any one of the items 1-2, wherein R¹ and R² are the same or different and represent peptides as defined above; particularly, R¹ and R² are the same or different and comprise a fragment corresponding to at least 2, 3, 4 or 5 contiguous amino acids contained in sequence SEQ ID NO: 1.
**5.** The compound of item 4, wherein R¹ and R² are the same or different and comprise the fragment SerLeuGlu; particularly, R¹ and R² are the same and consist of amino acid sequence SerLeuGlu.
**6.** The compound of item 3, wherein R¹ and R² are the same or different and comprise the amino acid Val; particularly, R¹ and R² are the same and consist of amino acid Val.
**7.** The compound of any one of the preceding items, wherein R¹ and R² comprise the same N-protecting group and it is one including an acyl (C=O) or ester group (-OCO-), such as acetyl, benzoyl, pivaloyl, tert-butyloxycarbonyl (BOC), N-Allyloxycarbonyl (Alloc), Benzyl chloroformate (Cbz), methylurethane or ethylurethane; particularly the N-protecting group is methylurethane or BOC.
**8.** The compound of any one of the preceding items, wherein R³ and R⁴ are the same, particularly hydrogen.
**9.** The compound of any one of the preceding items, wherein R⁵ to R¹² are the same or different and are independently selected from the group consisting of H, halogen, (C₁-C₁₀)alkyl, NO₂, OR¹³, SR¹⁴, NR¹⁵R¹⁶, and (+)NR¹⁷R¹⁸R¹⁹; particularly R⁵ to R¹² are hydrogen.
**10.** The compound of any one of the preceding items, wherein n is zero.
**11.** The compound of any one of the preceding items which is of formula (Id): 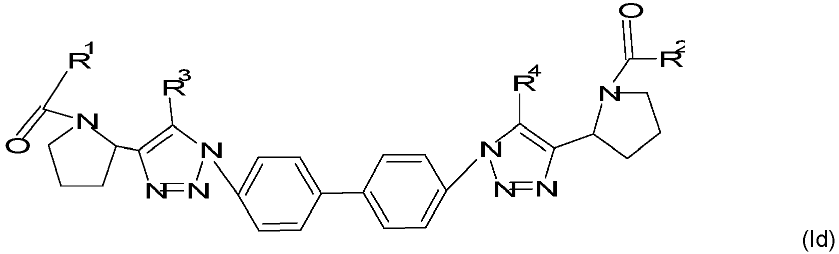 or a pharmaceutically acceptable salt or isomer thereof, wherein R¹, R², R³ and R⁴ are as defined in any of the preceding items.
**12.** The compound of item 11, wherein R¹ and R² are the same and correspond to the amino acid peptide SerLeuGlu or the amino acid Val, wherein the side chains of Ser and Glu residues are optionally blocked.
**13.** The compound of any one of the items 11-12, wherein R¹ and R² are N-terminal protected with the same group, which is selected from methyl urethane or Boc.
**14.** The compound of any one of the items 10-13, wherein R³ and R⁴ are the same, particularly they represent hydrogen.
**15.** A pharmaceutical composition comprising a therapeutically effective amount of the compound as defined in any of the preceding items together with one or more pharmaceutically acceptable excipients and/or carriers.
**16.** A compound as defined in any one of the preceding items 1 to 15 for use in therapy.
**17.** A compound as defined in any one of the preceding items 1 to 15 for use in the treatment of cancer, wherein there is an activation of Src in said cancer
**18.** The compound for use according to item 17, wherein said cancer is colorectal cancer.
**19.** A compound as defined in any one of the preceding items 1 to 15 for use in the treatment of thrombocytopenia, myelofibrosis, bleeding, platelet dysfunction with abnormal α-granules and bone pathologies.

## Claims

1. A compound of formula (I): 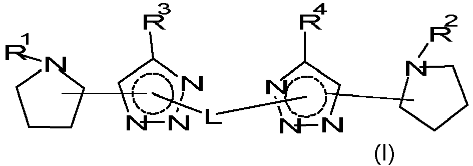
a pharmaceutically salt or isomer thereof,
wherein
R¹ and R² are the same or different and are selected from the group consisting of: a non-polar amino acid and a peptide, wherein
- the non-polar amino acid or peptide:
∘ is N-terminal protected,
∘ binds to the N atom of the pyrrolidinyl ring by its C-terminal end, forming an amide bond, and
∘ the lateral chain of the one or more amino acid(s) is optionally protected;
- the peptide comprises sequence SEQ ID NO: 1 or a fragment corresponding from 2 to 9 contiguous amino acids of sequence SEQ ID NO: 1;
R³ and R⁴ are the same or different and represent hydrogen, (C₁-C₅)alkyl, (C₂-C₅)alkenyl, (C₂-Cs)alkynyl, (C₁-C₅)alkyl substituted by one or more substituents Su₄, (C₂-C₅)alkenyl substituted by one or more substituents Su₅, or (C₂-C₅)alkynyl substituted by one or more substituents Su₆; and
L is of formula (II): 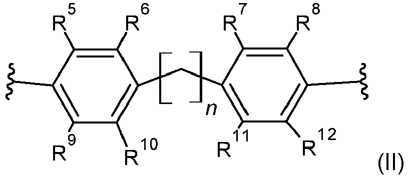
wherein
n is an entire number selected from 0 to 3, wherein when n=O, both rings are linked by a single bond;
R⁵ to R¹² are the same or different and are independently selected from the group consisting of: H, halogen, (C₁-C₁₀)alkyl, NO₂, OR¹³, SR¹⁴, NR¹⁵R¹⁶, and (+)NR¹⁷R¹⁸R¹⁹; or, alternatively,
R⁵ and R⁸ to R¹² are as defined above, and R⁶ and R⁷, together with the carbon atoms to which they are attached, form a ring system; or, alternatively,
R⁵ to R⁹ and R¹² are as defined above, and R¹⁰ and R¹¹ form, together with the carbon atoms to which they are attached, a ring system; or, alternatively,
R⁵, R⁸, R⁹ and R¹² are as defined above, R⁶ and R⁷ together with the carbon atoms to which they are attached form a ring system, and R¹⁰ and R¹¹ together with the carbon atoms to which they are attached form another ring system;
wherein the term "ring system" means a ring having from 6 to 10 members selected from CRₓ, C(RₓR_{y}), O, S, N, or NR_{y};
R¹³ and R¹⁴ represent hydrogen, (C₁-C₁₀)alkyl or (C₁-C₁₀) alkyl substituted by one or more substituents Su₇;
R¹⁵ to R¹⁹ are the same or different and are independently selected from the group consisting of: H, (C₁-C₁₀)alkyl, and (C₁-C₁₀)alkyl substituted by one or more substituents Su₈;
Rₓ and R_{y} represent hydrogen, (C₁-C₁₀)alkyl or (C₁-C₁₀) alkyl substituted by one or more substituents Su₉; and
Su₁ to Su₉ are independently selected from the group consisting of: halogen, cyano, nitro, (C₁-C₆)haloalkyl, (C₁-C₆)alkyl, (C₁-C₆)hydroxyalkyl, and (C₁-C₆)alkoxy.

2. The compound as defined in claim 1, which corresponds to formula (Ib) or (Ic): 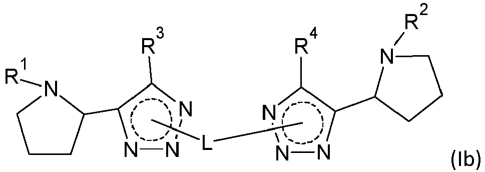 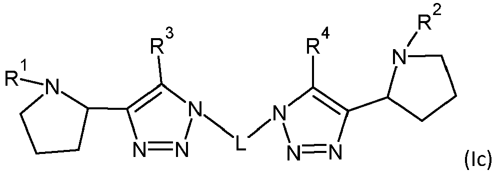 a pharmaceutically acceptable salt or isomer thereof, wherein R¹, R², R³, R⁴, and L are as defined in claim 1.

3. The compound of any one of the preceding claims, wherein R¹ and R² are the same and consist of amino acid Val.

4. The compound of any one of the claims 1-2, wherein R¹ and R² are the same and consist of amino acid sequence SerLeuGlu.

5. The compound of any one of the preceding claims, wherein R¹ and R² comprise the same N-protecting group and it is one including an acyl (C=O) or ester group (-OCO-), such as acetyl, benzoyl, pivaloyl, tert-butyloxycarbonyl (BOC), N-Allyloxycarbonyl (Alloc), Benzyl chloroformate (Cbz), methylurethane or ethylurethane; particularly the N-protecting group is methylurethane or BOC.

6. The compound of any one of the preceding claims, wherein n is zero.

7. The compound of any one of the preceding claims which is of formula (Id): 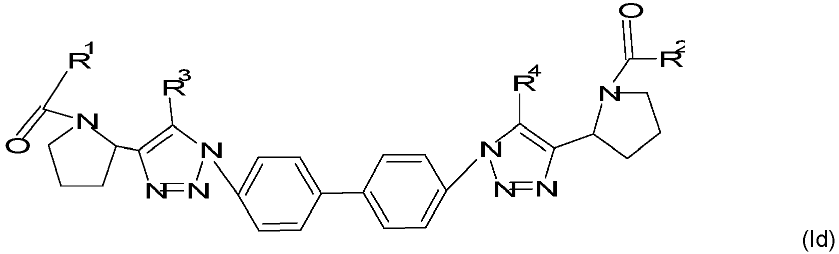 or a pharmaceutically acceptable salt or isomer thereof, wherein R¹, R², R³ and R⁴ are as defined in any of the preceding claims.

8. The compound of claim 7, wherein R¹ and R² are the same and correspond to the amino acid peptide SerLeuGlu or the amino acid Val, and wherein the side chains of Ser and Glu residues are optionally blocked.

9. The compound of claim 8, wherein R³ and R⁴ are hydrogen.

10. The compound of any one of the claims 7-9, wherein R¹ and R² are N-terminal protected with the same group, which is selected from methyl urethane or Boc.

11. The compound of any one of the preceding claims which is of formula (If): 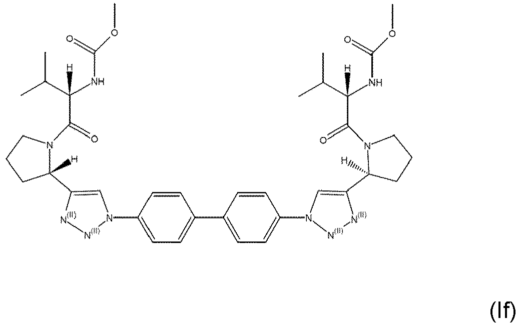 or a pharmaceutically acceptable salt or isomer thereof.

12. The compound of any one of the preceding claims which is of formula (Ig): 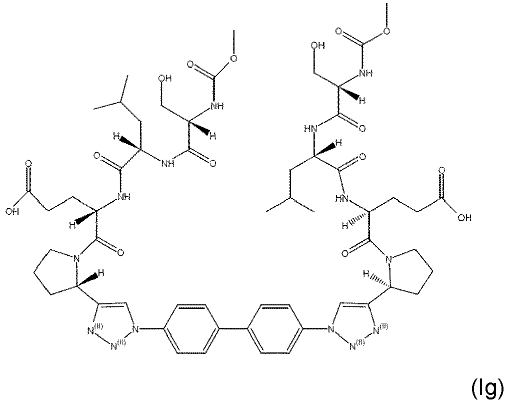 or a pharmaceutically acceptable salt or isomer thereof.

13. A pharmaceutical composition comprising a therapeutically effective amount of the compound as defined in any of the preceding claims together with one or more pharmaceutically acceptable excipients and/or carriers.

14. A compound as claimed in any one of the preceding claims 1 to 13 for use in therapy.

15. A compound as claimed in any one of the preceding claims 1 to 14 for use in the treatment of cancer, wherein there is an activation of Src in said cancer.
